# EUROPEAN PATENT SPECIFICATION

(11) **EP 0 393 457 B1**
(45) Date of publication and mention of the grant of the patent: **06.07.1994**
(21) Application number: 90106738.9
(22) Date of filing: 09.04.1990
(51) Int. Cl.: C07K 5/06, A61K 37/64

(54) **Proteinase inhibitor**
Protease-Hemmer
Inhibiteur de protéases

(30) Priority: 10.04.1989 JP 89904/89
(43) Date of publication of application: 24.10.1990
(73) Proprietor: SUNTORY LIMITED, Kita-ku, Osaka-shi, Osaka 530 (JP)
(72) Inventor: Higuchi, Naoki, Ikeda-shi, Osaka (JP); Saitoh, Masayuki, Ibaraki-shi, Osaka (JP); Shibata, Hiroshi, Nara-shi, Nara-ken (JP)
(74) Representative: Hansen, Bernd, Dr. Dipl.-Chem.

(56) References cited:
- DE-A- 1 958 383
- FR-A- 2 490 632
- FEBS LETTERS, vol. 195, nos. 1,2, January 1986, pages 265-268, Federation of European Biochemical Societies, Amsterdam, NL; G. CS.-SZABO et al.: "Specific inhibition of human granulocyte elastase with peptide aldhydes"
- BIOCHEMICAL AND BIOPHYSICAL RESEARCH COMMUNICATIONS, vol. 153, no. 3, 30th June 1988, pages 1201-1208, Academic Press, Inc., San Diego, US; T. TSUJINAKA et al.: "Synthesis of a new cell penetrating calpain inhibitor (calpeptin)"
- CHEMICAL ABSTRACTS, vol. 105, no. 25, 22nd December 1986, page 865, abstract no. 227326w, Columbus, Ohio, US
- CHEMICAL ABSTRACTS, vol. 103, no. 17, 28th October 1985, page 144, abstract no. 135650c, Columbus, Ohio, US; L. STEARDO et al.: "CCK26-33 degrading activity in brain and nonneural tissue: a metalloendopeptidase"

## Description

This invention relates to a novel N-substituted peptidyl aldehyde represented by the general formula (1) which will be given hereinafter. Furthermore, it relates to a cysteine proteinase inhibitor which comprises a compound of the general formula (1) as an active ingredient and exerts an intense enzyme inhibitory activity on cysteine proteinases, in particular, on papain, calpain and even on cathepsin.

It has been desired to develop a drug which specifically inhibits the activities of papain (E.C.3.4.22.2) and calpain (E.C.3.4.22.17) which are cysteine proteinases, since such a drug is useful as an anti-inflammatory agent. Calpain, which widely occurs in mammals and birds, is observed in cytosol. It is considered that abnormal activation of this enzyme causes serious diseases including muscular dystrophy and cataracts. Cathepsin (E.C.3.4.22.1) is a proteinase localized in lysosome. It is considered that abnormal activation of this enzyme causes cancer metastasis, amyotrophy and muscular dystrophy. Therefore, it has been desired to develop a drug which specifically inhibits the activities of calpain and cathepsin and thus is applicable as a remedy for muscular dystrophy, amyotrophy and cataracts or as a metastasis inhibitor. Attempts have been made to develop drugs useful for the above-mentioned purposes. As a result, a number of cysteine proteinase inhibitors have been found out hitherto [Shimizu B. et al., J. Antibiot., 25, 515 (1972); Japanese Patent Laid-Open No. 28990/1985; Japanese Patent Laid-Open No. 106600/1986; and Japanese Patent Laid-Open No. 103897/1986]. Tsujinaka, T. et al., Biochem. Biophys. Res. Comm., 153, 1988, 1201 - 1208 disclose the calpain inhibitor calpeptin (Z-Leu-nLeu-al). However, each of the known inhibitors required to be further improved in respect of activity and biomigration. Furthermore, it is urgently required to develop a compound which also exerts an intense inhibition activity on cathepsin.

The present invention provides a compound which exerts a strong inhibition activity on cysteine proteinases, in particular, cathepsin B and which shows excellent biomigration.

More particularly, the present invention provides a novel N-substituted peptidyl aldehyde represented by the following general formula (1): 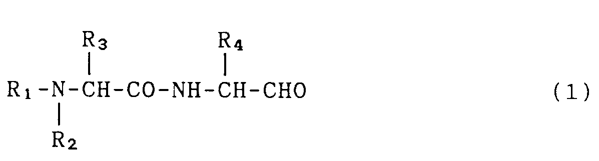
wherein R₁ represents a straight-chain or branched acyl group having 2 to 10 carbon atoms (e.g., octanoyl, caproyl or isovaleryl group), a branched, cyclic or polycyclic alkyloxycarbonyl group having 4 to 15 carbon atoms (e.g., t-butyloxycarbonyl, adamantyloxycarbonyl or isobornyloxycarbonyl group), a benzyloxycarbonyl group optionally substituted with, e.g., a halogen atom, a nitro group or a methoxy group, a 2,2,2-trichloroethyloxycarbonyl group, a 2-(trimethylsilyl)ethyloxycarbonyl group, a p-toluenesulfonyl group, an o-nitrophenylsulfenyl group, a diphenylphosphonothioyl group, a triphenylmethyl group or a 2-benzoyl-1-methylvinyl group;
R₂ represents a hydrogen atom; or R₁ and R₂ may together form a phthaloyl group;
R₃ represents an isobutyl group or a n-butyl group and the above-mentioned R₁ can be an unsubstituted-benzyloxycarbonyl group provided that R₃ is a n-butyl group; and
R₄ represents a n-butyl group;
which exerts a strong activity of inhibiting cathepsin B, calpain or papain.

The peptide derivative compound of the present invention represented by the general formula (1) strongly inhibits papain, calpain and cathepsin B. Thus, it is expected that this compound will further inhibit other cysteine proteinases, for example, cathepsin H or L. Furthermore, it is expected that this compound would be useful in the treatment of inflammation, cataracts, epidermolysis bullosa and pemphigus which might be caused by these cysteine proteinases. Furthermore, an affinity column prepared by coupling the compound of the present invention on an appropriate support is available in the purification of cysteine proteinases. In addition, it is expected that the compound of the present invention would be available as a reagent in the fields of biochemistry and enzymology.

The compound of the present invention may be prepared in the following manner.

First, a compound of the general formula (1) wherein a group represented by R₁ or a group formed by R₁ together with R₂ is stable in treatment with a base or reduction with a reducing agent may be prepared as follows. A compound represented by the following general formula (2): 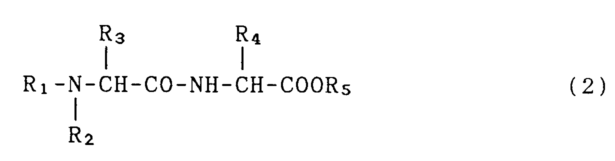
wherein R₁, R₂, R₃ and R₄ are as defined above regarding the general formula (1); and
R₅ represents a lower alkyl group;
is reduced by use of a reducing agent in an organic solvent so as to give an alcohol compound. Next, the obtained alcohol compound is oxidized by use of an oxidizing agent to thereby give the aldehyde. Alternatively, a compound represented by the following general formula (3): 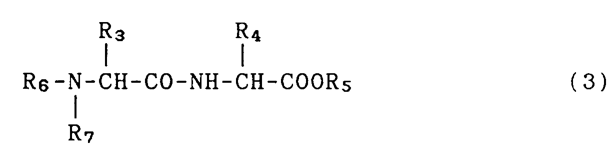
wherein R₃, R₄ and R₅ are as defined above regarding the above general formula (2);
R₆ represents an amino-protecting group which is stable to a treatment with a base or reduction by use of a reducing agent; and
R₇ represents a hydrogen atom; or
R₆ and R₇ together form an amino-protecting group which is stable to a treatment with a base or reduction by use of a reducing agent;
can be used as the starting compound. The protecting group represented by R₆ or formed by R₆ together with R₇ is removed by an appropriate method. A desired group R₁ or a desired group formed by R₁ together with R₂ is then introduced to the amino group to give a compound represented by the general formula (2), which is then converted to a compound of the general formula (1) as described above.

A compound represented by the general formula (1) wherein a group represented by R₁ or a group formed by R₁ together with R₂ is unstable into a treatment with a base or reduction by use of a reducing agent may be prepared as follows. A compound represented by the following general formula (3): 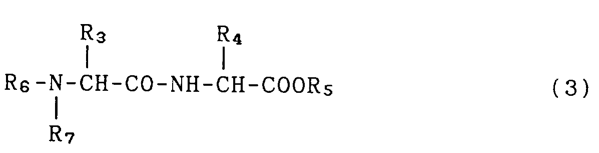
wherein R₃, R₄ and R₅ are as defined above regarding the above general formula (2);
R₆ represents an amino-protecting group which is stable to a treatment with a base or reduction by use of a reducing agent; and
R₇ represents a hydrogen atom; or
R₆ and R₇ together form an amino-protecting group which is stable to a treatment with a base or reduction by use of a reducing agent;
is reduced by use of a reducing agent in an organic solvent so as to give an alcohol compound. Next, the protecting group R₆ or the one formed by R₆ together with R₇ is removed by an appropriate method. Then a desired R₁ group or the one formed by R₁ together with R₂ is introduced into the amino group by an appropriate method. The alcohol moiety of the compound thus obtained is then oxidized in the same manner as the one described above so as to give the aldehyde. Thus the aimed compound can be readily obtained.

### Examples

To further illustrate the present invention, the following enzyme inhibition activity tests and Examples will be given. It is needless to say, however, that the technical scope of the present invention is not restricted thereby.

### Example 1

### Production of N-octanoyl-L-leucyl-L-norleucinal [in general formula (1), R₁ = CH₃(CH₂)₆CO-, R₂ = H-, R₃ = (CH₃)₂CHCH₂-, R₄ = CH₃(CH₂)₃-]:

### (a) Production of N-octanoyl-L-leucine

2.6 g of L-leucine was dissolved in 20 ml of 1 N sodium hydroxide. 3.3 ml of octanoyl chloride and 20 ml of 1 N sodium hydroxide were added thereto under ice-cooling and the mixture so formed was stirred at room temperature for 8 hours. After the completion of the reaction, the reaction mixture was washed with ether. 5 N hydrochloric acid was added to the aqueous phase to thereby lower the pH value to 2 or below followed by extracting with ethyl acetate. The extract was dried over anhydrous sodium sulfate. After distilling off the solvent under reduced pressure, the residue was recrystallized from a solvent mixture of ethyl acetate and hexane. Thus, 4.3 g of the titled N-octanoyl-L-leucine was obtained in the form of crystals.

### (b) Production of N-octanoyl-L-leucyl-L-norleucine methyl ester

2.6 g of the N-octanoyl-L-leucine obtained in the above step (a) and 1.8 g of L-norleucine methyl ester hydrochloride were dissolved in 50 ml of dry dimethylformamide. Next, 1.6 g of diethyl cyanophosphonate was added thereto. 2.0 g of triethylamine was further added to the obtained solution under ice-cooling and the resulting mixture was stirred at room temperature for 12 hours. After the completion of the reaction, 200 ml of water was added to the reaction mixture. Then it was extracted with ether. The organic phase was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 2.2 g of the titled N-octanoyl-L-leucyl-L-norleucine methyl ester was obtained in the form of an oily product.

### (c) Production of N-octanoyl-L-leucyl-L-norleucinol

2.2 g of the N-octanoyl-L-leucyl-L-norleucine methyl ester obtained in the above step (b) and 1.0 g of sodium borohydride were suspended in 20 ml of t-butyl alcohol and then heated under reflux in a nitrogen atmosphere at 90°C. Next, 8 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 1.7 g of the titled N-octanoyl-L-leucyl-L-norleucinol was obtained in the form of crystals.

### (d) Production of N-octanoyl-L-leucyl-L-norleucinal

1.7 g of the N-octanoyl-L-leucyl-L-norleucinol obtained in the above step (c) and 2.0 g of triethylamine were dissolved in 20 ml of dry dimethylsulfoxide. Then a solution of 3.0 g of sulfur trioxide/pyridine complex dissolved in 20 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 1.0 g of the target compound N-octanoyl-L-leucyl-L-norleucinal was obtained in the form of crystals.

### Example 2

### Production of N-hexanoyl-L-leucyl-L-norleucinal [in general formula (1), R₁ = CH₃(CH₂)₄CO-, R₂ = H-, R₃ = (CH₃)₂CHCH₂-, R₄ = CH₃(CH₂)₃-]:

### (a) Production of N-hexanoyl-L-leucine

2.6 g of L-leucine was dissolved in 20 ml of 1 N sodium hydroxide. 3.0 g of hexanoyl chloride and 20 ml of 1 N sodium hydroxide were added thereto under ice-cooling and the mixture so formed was stirred at room temperature for 8 hours. After the completion of the reaction, the reaction mixture was washed with ether. 5 N hydrochloric acid was added to the aqueous phase to thereby lower the pH value to 2 or below followed by extracting with ethyl acetate. The extract was dried over anhydrous sodium sulfate. After distilling off the solvent under reduced pressure, the residue was recrystallized from a solvent mixture of ethyl acetate and hexane. Thus, 2.8 g of the titled N-hexanoyl-L-leucine was obtained in the form of crystals.

### (b) Production of N-hexanoyl-L-leucyl-L-norleucine methyl ester

2.7 g of the N-hexanoyl-L-leucine obtained in the above step (a) and 2.2 g of L-norleucine methyl ester hydrochloride were dissolved in 50 ml of dry dimethyformamide. Next, 2.0 g of diethyl cyanophosphonate was added thereto. 2.0 g of triethylamine was further added to the obtained solution under ice-cooling and the resulting mixture was stirred at room temperature for 12 hours. After the completion of the reaction, 200 ml of water was added to the reaction mixture. Then it was extracted with ether. The organic phase was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 2.2 g of the titled N-hexanoyl-L-leucyl-L-norleucine methyl ester was obtained in the form of an oily product.

### (c) Production of N-hexanoyl-L-leucyl-L-norleucinol

2.2 g of the N-hexanoyl-L-leucyl-L-norleucine methyl ester obtained in the above step (b) and 1.0 g of sodium borohydride were suspended in 20 ml of t-butyl alcohol and then heated under reflux in a nitrogen atmosphere at 90°C. Next, 8 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was recrystallized from a solvent mixture of ethyl acetate and hexane. Thus 1.6 g of the titled N-hexanoyl-L-leucyl-L-norleucinol was obtained in the form of crystals.

### (d) Production of N-hexanoyl-L-leucyl-L-norleucinal

1.6 g of the N-hexanoyl-L-leucyl-L-norleucinol obtained in the above step (c) and 2.0 g of triethylamine were dissolved in 15 ml of dry dimethylsulfoxide. Then a solution of 3.0 g of sulfur trioxide/pyridine complex dissolved in 15 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate under reduced pressure, the residue was purified by medium pressure column chromatography by use of silica gel. Thus 1.0 g of the target compound N-hexanoyl-L-leucyl-L-norleucinal was obtained in the form of powder.

### Example 3

### Production of N-isovaleryl-L-leucyl-L-norleucinal [in general formula (1), R₁ = (CH₃)₂CHCH₂CO-, R₂ = H-, R₃ = (CH₃)₂CHCH₂-, R₄ = CH₃(CH₂)₃-]:

### (a) Production of N-isovaleryl-L-leucine

5.2 g of L-leucine was dissolved in 40 ml of 1 N sodium hydroxide. 2.4 g of isovaleryl chloride and 40 ml of 1 N sodium hydroxide were added thereto under ice-cooling and the mixture so formed was stirred at room temperature for 8 hours. After the completion of the reaction, the reaction mixture was washed with either. 5 N hydrochloric acid was added to the aqueous phase to thereby lower the pH value to 2 or below followed by extracting with ethyl acetate. The extract was dried over anhydrous sodium sulfate. After distilling off the solvent under reduced pressure, the residue was recrystallized from a solvent mixture of ethyl acetate and hexane. Thus 2.6 g of the titled N-isovaleryl-L-leucine was obtained in the form of crystals.

### (b) Production of N-isovaleryl-L-leucyl-L-norleucine methyl ester

2.6 g of the N-isovaleryl-L-leucine obtained in the above step (a) and 2.2 g of L-norleucine methyl ester hydrochloride were dissolved in 50 ml of dry dimethylformamide. Next, 2.0 g of diethyl cyanophosphonate was added thereto. 2.0 g of triethylamine was further added to the obtained solution under ice-cooling and the resulting mixture was stirred at room temperature for 12 hours. After the completion of the reaction, 200 ml of water was added to the reaction mixture. Then it was extracted with ether. The organic phase was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 1.6 g of the titled N-isovaleryl-L-leucyl-L-norleucine methyl ester was obtained in the form of an oily product.

### (c) Production of N-isovaleryl-L-leucyl-L-norleucinol

1.6 g of the N-isovaleryl-L-leucyl-L-norleucine methyl ester obtained in the above step (b) and 1.0 g of sodium borohydride were suspended in 30 ml of t-butyl alcohol and then heated under reflux in a nitrogen atmosphere at 90°C. Next, 5 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was recrystallized from a solvent mixture of ethyl acetate and hexane. Thus 1.2 g of the titled N-isovaleryl-L-leucyl-L-norleucinol was obtained in the form of crystals.

### (d) Production of N-isovaleryl-L-leucyl-L-norleucinal

1.0 g of the N-isovaleryl-L-leucyl-L-norleucinol obtained in the above step (c) and 2.0 g of triethylamine were dissolved in 15 ml of dry dimethylsulfoxide. Then a solution of 2.0 g of sulfur trioxide/pyridine complex dissolved in 15 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 0.7 g of the target compound N-isovaleryl-L-leucyl-L-norleucinal was obtained in the form of crystals.

### Example 4

### Production of N-t-butyloxycarbonyl-L-leucyl-L-norleucinal [in general formula (1), R₁ = (CH₃)₃COCO-, R₂ = H-, R₃ = (CH₃)₂CHCH₂-, R₄ = CH₃(CH₂)₃-]:

### (a) Production of N-t-butyloxycarbonyl-L-leucyl-L-norleucine methyl ester

2.5 g of N-t-butyloxycarbonyl-L-leucine monohydrate was dissolved in 50 ml of dry methylene chloride. Then 1.2 g of 1-ethyl-3-(3-dimethylaminopropyl)carbodiimide hydrochloride was added thereto. To the solution so formed, was added a solution of 1.8 g of L-norleucine methyl ester hydrochloride and 1.2 g of triethylamine dissolved in 50 ml of dry methylene chloride. The resulting mixture was stirred at room temperature for 6 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 4.0 g of the titled N-t-butyloxycarbonyl-L-leucyl-L-norleucine methyl ester was obtained in the form of crystals.

### (b) Production of N-t-butyloxycarbonyl-L-leucyl-L-norleucinol

3.0 g of the N-t-butyloxycarbonyl-L-leucyl-L-norleucine methyl ester obtained in the above step (a) and 3.0 g of sodium borohydride were suspended in 60 ml of t-butyl alcohol and then heated under reflux in a nitrogen atmosphere at 90°C. Next, 10 ml of absolute methanol was added thereto dropwise under reflux. After the completion of the addition, the mixture was stirred under reflux for minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.0 g of the titled N-t-butyloxycarbonyl-L-leucyl-L-norleucinol was obtained in the form of crystals.

### (c) Production of N-t-butyloxycarbonyl-L-leucyl-L-norleucinal

1.0 g of the N-t-butyloxycarbonyl-L-leucyl-L-norleucinol obtained in the above step (b) and 1.2 g of triethylamine were dissolved in 12 ml of dry dimethylsulfoxide. Then a solution of 2.0 g of sulfur trioxide/pyridine complex dissolved in 12 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 0.5 g of the target compound N-t-butyloxycarbonyl-L-leucyl-L-norleucinal was obtained in the form of crystals.

### Example 5

### Production of N-adamantyloxycarbonyl-L-leucyl-L-norleucinal [in general formula (1), R₁ = tricyclo[3.3.1.13,7]decane-OCO-, R₂ = H-, R₃ = (CH₃)₂CHCH₂-, R₄ = CH₃(CH₂)₃-]:

### (a) Production of N-adamantyloxycarbonyl-L-leucine

2.6 g of L-leucine and 7.0 g of potassium carbonate were dissolved in 100 ml of water. 4.0 g of adamantyloxycarbonyl fluoride was added thereto under ice-cooling and the mixture so formed was stirred at room temperature for 3 hours. After the completion of the reaction, the reaction mixture was washed with ether. 50% phosphoric acid was added to the aqueous phase to thereby lower the pH value to 2 or below followed by extracting with ether. The extract was dried over anhydrous sodium sulfate. After distilling off the solvent under reduced pressure, the residue was recrystallized from a solvent mixture of ethyl acetate and hexane. Thus 3.6 g of the titled N-adamantyloxycarbonyl-L-leucine was obtained in the form of crystals.

### (b) Production of N-adamantyloxycarbonyl-L-leucyl-L-norleucine methyl ester

3.1 g of the N-adamantyloxycarbonyl-L-leucine obtained in the above step (a) was dissolved in 50 ml of dry methylene chloride. 1.2 g of 1-ethyl-3-(3-dimethylaminopropyl)carbodiimide hydrochloride was added thereto. To the solution so formed, was added a solution of 1.8 g of L-norleucine methyl ester hydrochloride and 1.2 g of triethylamine dissolved in 50 ml of dry methylene chloride. The mixture was stirred at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 2.8 g of the titled N-adamantyloxycarbonyl-L-leucyl-L-norleucine methyl ester was obtained in the form of an oily product.

### (c) Production of N-adamantyloxycarbonyl-L-leucyl-L-norleucinol

2.6 g of the N-adamantyloxycarbonyl-L-leucyl-L-norleucine methyl ester obtained in the above step (b) and 1.0 g of sodium borohydride were suspended in 50 ml of t-butyl alcohol and then heated under reflux in a nitrogen atmosphere at 90°C. Next, 10 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.2 g of the titled N-adamantyloxycarbonyl-L-leucyl-L-norleucinol was obtained in the form of a powder.

### (d) Production of N-adamantyloxycarbonyl-L-leucyl-L-norleucinal

1.0 g of the N-adamantyloxycarbonyl-L-leucyl-L-norleucinol obtained in the above step (c) and 1.0 g of triethylamine were dissolved in 10 ml of dry dimethylsulfoxide. Then a solution of 1.6 g of sulfur trioxide/pyridine complex dissolved in 12 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was purified by reverse phase medium pressure column chromatography by use of octadecyl silane. Thus 0.8 g of the target compound N-adamantyloxycarbonyl-L-leucyl-L-norleucinal was obtained in the form of a powder.

### Example 6

### Production of N-p-chlorobenzyloxycarbonyl-L-leucyl-L-norleucinal [in general formula (1), R₁ = (4-Cl)C₆H₄CH₂OCO-, R₂ = H-, R₃ = (CH₃)₂CHCH₂-, R₄ = CH₃(CH₂)₃-]:

### (a) Production of N-p-chlorobenzyloxycarbonyl-L-leucine dicyclohexylamine salt

3.9 g of L-leucine and 4.2 g of potassium carbonate were dissolved in 100 ml of water. A solution of 6.2 g of p-chlorobenzyloxycarbonyl chloride dissolved in 10 ml of dioxane was added thereto under ice-cooling. The mixture so formed was stirred at room temperature for 8 hours. After the completion of the reaction, the reaction mixture was washed with ether. 5 N hydrochloric acid was added to the aqueous phase to thereby lower the pH value to 2 or below followed by extracting with ethyl acetate. The extract was dried over anhydrous sodium sulfate. After concentrating the solvent under reduced pressure, 6.0 ml of dicyclohexylamine was added thereto. Thus 9.5 g of the titled N-p-chlorobenzyloxycarbonyl-L-leucine dicyclohexylamine salt was obtained in the form of crystals.

### (b) Production of N-p-chlorobenzyloxycarbonyl-L-leucyl-L-norleucine methyl ester

4.8 g of the N-p-chlorobenzyloxycarbonyl-L-leucine dicyclohexylamine salt obtained in the above step (a) was suspended in 100 ml of ethyl acetate and washed with a 10% aqueous solution of citric acid. The organic phase was dried over anhydrous sodium sulfate. After distilling off the solvent, the residue was dissolved in 50 ml of methylene chloride. 2.2 g of 1-ethyl-3-(3-dimethylaminopropyl)carbodiimide hydrochloride was added thereto. To the solution thus formed, was added a solution of 1.8 g of L-norleucine methyl ester hydrochloride and 1.2g of triethylamine dissolved in 50 ml of dry methylene chloride followed by stirring at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 3.0 g of the titled N-p-chlorobenzyloxycarbonyl-L-leucyl-L-norleucine methyl ester was obtained in the form of crystals.

### (c) Production of N-p-chlorobenzyloxycarbonyl-L-leucyl-L-norleucinol

3.0 g of the N-p-chlorobenzyloxycarbonyl-L-leucyl-L-norleucine methyl ester obtained in the above step (b) and 1.0 g of sodium borohydride were suspended in 40 ml of t-butyl alcohol and then heated under reflux in a nitrogen atmosphere at 90°C. Next, 10 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.2 g of the titled N-p-chlorobenzyloxycarbonyl-L-leucyl-L-norleucinol was obtained in the form of an oily product.

### (d) Production of N-p-chlorobenzyloxycarbonyl-L-leucyl-L-norleucinal

1.0 g of the N-p-chlorobenzyloxycarbonyl-L-leucyl-L-norleucinol obtained in the above step (c) and 1.2 g of triethylamine were dissolved in 10 ml of dry dimethylsulfoxxide. Then a solution of 2.0 g of sulfur trioxide/pyridine complex dissolved in 10 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 0.7 g of the target compound N-p-chlorobenzyloxycarbonyl-L-leucyl-L-norleucinal was obtained in the form of crystals.

### Example 7

### Production of N-p-methoxybenzyloxycarbonyl-L-leucyl-L-norleucinal [in general formula (1), R₁ = (4-CH₃O)C₆H₄CH₂OCO-, R₂ = H-, R₃ = (CH₃)₂CHCH₂-, R₄ = CH₃(CH₂)₃-]:

### (a) Production of N-p-methoxybenzyloxycarbonyl-L-leucine dicyclohexylamine salt

3.9 g of L-leucine and 7.0 g of triethylamine were dissolved in 15 ml of water. A solution of 9.1 g of p-methoxybenzyl-S-4,6-dimethylpyrimidin-2-ylthiocarbonate dissolved in 20 ml of dioxane was added thereto under ice-cooling. The mixture so formed was stirred at room temperature for 5 hours. After the completion of the reaction, 150 ml of water was added to the reaction mixture followed by washing with ethyl acetate. 5 N hydrochloric acid was added to the aqueous phase to thereby lower the pH value to 2 or below followed by extracting with ethyl acetate. The extract was dried over sodium sulfate. After distilling off the solvent under reduced pressure, 6.0 ml of dicyclohexylamine was added to the concentrated solution. Thus 9.0 g of the titled N-p-methoxybenzyloxycarbonyl-L-leucine dicyclohexylamine salt was obtained in the form of crystals.

### (b) Production of N-p-methoxybenzyloxycarbonyl-L-leucyl-L-norleucine methyl ester

4.7 g of the N-p-methoxybenzyloxycarbonyl-L-leucine dicyclohexylamine salt obtained in the above step (a) was suspended in 100 ml of ethyl acetate and washed with a 10% aqueous solution of citric acid. The organic phase was dried over anhydrous sodium sulfate. After distilling off the solvent, the residue was dissolved in 50 ml of dry methylene chloride. 2.2 g of 1-ethyl-3-(3-dimethylaminopropyl)carbodiimide hydrochloride was added thereto. To the solution thus formed, was added a solution of 1.8 g of L-norleucine methyl ester hydrochloride and 1.2 g of triethylamine dissolved in 50 ml of dry methylene chloride followed by stirring at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 3.0 g of the titled N-p-methoxybenzyloxycarbonyl-L-leucyl-L-norleucine methyl ester was obtained in the form of crystals.

### (c) Production of N-p-methoxybenzyloxycarbonyl-L-leucyl-L-norleucinol

2.0 g of the N-p-methoxybenzyloxycarbonyl-L-leucyl-L-norleucine methyl ester obtained in the above step (b) and 1.0 g of sodium borohydride were suspended in 40 ml of t-butyl alcohol and then heated under reflux in a nitrogen atmosphere at 90°C. Next, 10 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.0 g of the titled N-p-methoxybenzyloxycarbonyl-L-leucyl-L-norleucinol was obtained in the form of crystals.

### (d) Production of N-p-methoxybenzyloxycarbonyl-L-leucyl-L-norleucinal

1.0 g of the N-p-methoxybenzyloxycarbonyl-L-leucyl-L-norleucinol obtained in the above step (c) and 1.2 g of triethylamine were dissolved in 10 ml of dry dimethylsulfoxxide. Then a solution of 2.0 g of sulfur trioxide/pyridine complex dissolved in 10 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous solution sulfate. After distilling off the ethyl acetate, the residue was recrystallized from a solvent mixture of ethyl acetate and hexane. Thus 0.7 g of the target compound N-p-methoxybenzyloxycarbonyl-L-leucyl-L-norleucinal was obtained in the form of crystals.

### Example 8

### Production of N-p-nitrobenzyloxycarbonyl-L-leucyl-L-norleucinal [in general formula (1), R₁ = (4-NO₂)C₆H₄CH₂OCO-, R₂ = H-, R₃ = (CH₃)₂CHCH₂-, R₄ = CH₃(CH₂)₃-]:

### (a) Production of N-benzyloxycarbonyl-L-leucyl-L-norleucine methyl ester

7.4 g of N-benzyloxycarbonyl-L-leucine was dissolved in 100 ml of dry methylene chloride and 8.2 g of 1-ethyl-3-(3-dimethylaminopropyl)carbodiimide hydrochloride was added thereto. To the solution so formed, was added a solution of 7.4 g of L-norleucine methyl ester hydrochloride and 4.2 g of triethylamine in 100 ml of dry methylene chloride. The resulting mixture was stirred at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and dried over sodium sulfate. After distilling off the solvent, the residue was purified by medium pressure column chromatography by use of silica gel. Thus 12 g of the titled N-benzyloxycarbonyl-L-leucyl-L-norleucine methyl ester was obtained in the form of crystals.

### (b) Production of N-benzyloxycarbonyl-L-leucyl-L-norleucinol

10 g of the N-benzyloxycarbonyl-L-leucyl-L-norleucine methyl ester obtained in the above step (a) and 3.0 g of sodium borohydride were suspended in 100 ml of t-butyl alcohol and then heated under reflux in a nitrogen atmosphere at 90°C. Next, 20 ml of absolute methanol was added thereto dropwise under reflux. After the completion of the addition, the mixture was stirred under reflux for 30 minutes and then cooled to room temperature. 100 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 8.0 g of the titled N-benzyloxycarbonyl-L-leucyl-L-norleucinol was obtained in the form of crystals.

### (c) Production of N-p-nitrobenzyloxycarbonyl-L-leucyl-L-norleucinol

2.5 g of the N-benzyloxycarbonyl-L-leucyl-L-norleucinol obtained in the above step (b) was dissolved in 50 ml of ethanol. Then a catalytic amount of palladium carbon was added thereto and the mixture so formed was stirred under a hydrogen atmosphere for 3 hours. After the completion of the reaction, the palladium carbon was filtered off and the solvent was distilled off from the filtrate under reduced pressure. Thus L-leucyl-L-norleucinol was quantitatively obtained. Next, 20 ml of water and 1.5 g of sodium hydrogencarbonate were added thereto. Further, a solution of 1.5 g of p-nitrobenzyloxycarbonyl chloride dissolved in 10 ml of ether was added thereto followed by stirring at room temperature for 3 hours. After the completion of the reaction, the mixture was thrice extracted with ethyl acetate, washed with a saturated aqueous solution of NaCl and then dried over anhydrous magnesium sulfate. After distilling off the ethyl acetate, the residue was recrystallized from a solvent mixture of ethyl acetate and hexane. Thus 2.0 g of the titled N-p-nitrobenzyloxycarbonyl-L-leucyl-L-norleucinol was obtained in the form of crystals.

### (d) Production of N-p-nitrobenzyloxycarbonyl-L-leucyl-L-norleucinal

1.0 g of the N-p-nitrobenzyloxycarbonyl-L-leucyl-L-norleucinol obtained in the above step (c) and 1.2 g of triethylamine were dissolved in 10 ml of dry dimethylsulfoxide. Then a solution of 2.0 g of sulfur trioxide/pyridine complex dissolved in 10 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 0.8 g of the target compound N-p-nitrobenzyloxycarbonyl-L-leucyl-L-norleucinal was obtained in the form of crystals.

### Example 9

### Production of N-o-chlorobenzyloxycarbonyl-L-leucyl-L-norleucinal [in general formula (1), R₁ = (2-Cl)C₆H₄CH₂OCO-, R₂ = H-, R₃ = (CH₃)₂CHCH₂-, R₄ = CH₃(CH₂)₃-]:

### (a) Production of N-o-chlorobenzyloxycarbonyl-L-leucine dicyclohexylamine salt

4.0 g of L-leucine ethyl ester hydrochloride and 2.1 g of triethylamine were dissolved in 100 ml of dry tetrahydrofuran. Then 5.0 g of N-(2-chlorobenzyloxycarbonyl)oxysuccinimide was added thereto under ice-cooling. The mixture so formed was stirred at room temperature for 12 hours. After the completion of the reaction, the solvent was distilled off under reduced pressure and 50 ml of water was added to the residue. After extracting with ethyl acetate, the extract was dried over sodium sulfate. The solvent was distilled off under reduced pressure and the residue was purified with medium pressure column chromatography by use of silica gel. Thus 7.0 g of N-o-chlorobenzyloxycarbonyl-L-leucine ethyl ester was obtained in the form of an oily product. Next, this product was dissolved in 20 ml of methanol and 30 ml of 1 N sodium hydroxide and 50 ml of water were added thereto. The mixture so formed was heated to 80°C under stirring for 1 hour. After the completion of the reaction, the reaction mixture was washed with ethyl acetate. 5 N hydrochloric acid was added to the aqueous phase to thereby lower the pH value to 2 or below followed by extracting with ethyl acetate. The organic phase was dried over anhydrous sodium sulfate and concentrated under reduced pressure. After adding 6.0 g of dicyclohexylamine, 5.5 g of the titled N-o-chlorobenzyloxycarbonyl-L-leucine dicyclohexylamine salt was obtained in the form of crystals.

### (b) Production of N-o-chlorobenzyloxycarbonyl-L-leucyl-L-norleucine methyl ester

4.8 g of the N-o-chlorobenzyloxycarbonyl-L-leucine dicyclohexylamine salt obtained in the above step (a) was suspended in 100 ml of ethyl acetate and washed with a 10% aqueous solution of citric acid. The organic phase was dried over sodium sulfate. After distilling off the solvent, the residue was dissolved in 50 ml of methylene chloride. 2.2 g of 1-ethyl-3-(3-dimethylaminopropyl)carbodiimide hydrochloride was added thereto. To the solution thus formed, was added a solution of 1.8 g of L-norleucine methyl ester hydrochloride and 1.2 g of triethylamine dissolved in 50 ml of dry methylene chloride followed by stirring at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 3.0 g of the titled N-o-chlorobenzyloxycarbonyl-L-leucyl-L-norleucine methyl ester was obtained in the form of crystals.

### (c) Production of N-o-chlorobenzyloxycarbonyl-L-leucyl-L-norleucinol

3.0 g of the N-o-chlorobenzyloxycarbonyl-L-leucyl-L-norleucine methyl ester obtained in the above step (b) and 1.0 g of sodium borohydride were suspended in 50 ml of t-butyl alcohol and then heated under reflux in a nitrogen atmosphere at 90°C. Next, 10 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.0 g of the titled N-o-chlorobenzyloxycarbonyl-L-leucyl-L-norleucinol was obtained in the form of crystals.

### (d) Production of N-o-chlorobenzyloxycarbonyl-L-leucyl-L-norleucinal

1.0 g of the N-o-chlorobenzyloxycarbonyl-L-leucyl-L-norleucinol obtained in the above step (c) and 1.2 g of triethylamine were dissolved in 15 ml of dry dimethylsulfoxide. Then a solution of 2.0 g of sulfur trioxide/pyridine complex dissolved in 15 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 0.8 g of the target compound N-o-chlorobenzyloxycarbonyl-L-leucyl-L-norleucinal was obtained in the form of crystals.

### Example 10

### Production of N-(2,2,2-trichloroethyl)oxycarbonyl-L-leucyl-L-norleucinal [in general formula (1), R₁ = CCl₃CH₂OCO-, R₂ = H-, R₃ = (CH₃)₂CHCH₂-, R₄ = CH₃(CH₂)₃-]:

### (a) Production of N-benzyloxycarbonyl-L-leucyl-norleucine methyl ester

7.4 g of N-benzyloxycarbonyl-L-leucine was dissolved in 100 ml of dry methylene chloride and 8.2 g of 1-ethyl-3-(3-diethyl-aminopropyl)carbodiimide hydrochloride was added thereto. To the solution so formed, was added a solution of 7.4 g of L-norleucine methyl ester hydrochloride and 4.2 g of triethylamine dissolved in 100 ml of dry methylene chloride. The resulting mixture was stirred at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and dried over sodium sulfate. After distilling off the solvent, the residue was purified by medium pressure column chromatography by use of silica gel. Thus 12 g of the titled N-benzyloxycarbonyl-L-leucyl-L-norleucine methyl ester was obtained in the form of crystals.

### (b) Production of N-benzyloxycarbonyl-L-leucyl-L-norleucinol

10 g of the N-benzyloxycarbonyl-L-leucyl-L-norleucine methyl ester obtained in the above step (a) and 3.0 g of sodium borohydride were suspended in 100 ml of t-butyl alcohol and then heated under reflux in a nitrogen atmosphere at 90°C. Next, 20 ml of absolute methanol was added thereto dropwise under reflux. After the completion of the addition, the mixture was stirred under reflux for 30 minutes and then cooled to room temperature. 100 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 8.0 g of the titled N-benzyloxycarbonyl-L-leucyl-L-norleucinol was obtained in the form of crystals.

### (c) Production of N-2,2,2-trichloroethyl-oxycarbonyl-L-leucyl-L-norleucinol

2.5 g of the N-benzyloxycarbonyl-L-leucyl-L-norleucinol obtained in the above step (b) was dissolved in 50 ml of ethanol. Then a catalytic amount of palladium carbon was added thereto and the mixture so formed was stirred under a hydrogen atmosphere for 3 hours. After the completion of the reaction, the palladium carbon was filtered off and the solvent was distilled off from the filtrate under reduced pressure. Thus L-leucyl-L-norleucinol was quantitatively obtained. Next, 20 ml of water and 2.2 g of sodium hydrogencarbonate were added thereto. Further, a solution of 1.5 g of 2,2,2-trichloroethyloxycarbonyl chloride in 10 ml of ether was added thereto followed by stirring at room temperature for 3 hours. After the completion of the reaction, the mixture was thrice extracted with ethyl acetate, washed with a saturated aqueous solution of NaCl and then dried over anhydrous magnesium sulfate. After distilling off the ethyl acetate, the residue was purified by medium pressure column chromatography by use of silica gel. Thus 1.0 g of the titled N-2,2,2-trichloroethyl-oxycarbonyl-L-leucyl-L-norleucinol was obtained in the form of crystals.

### (d) Production of N-2,2,2-trichloroethyl-oxycarbonyl-L-leucyl-L-norleucinal

0.7 g of the N-2,2,2-trichloroethyl-oxycarbonyl-L-leucyl-L-norleucinol obtained in the above step (c) and 1.0 g of triethylamine were dissolved in 10 ml of dry dimethylsulfoxide Then a solution of 1.5 g of sulfur trioxide/pyridine complex dissolved in 10 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 0.5 g of the target compound N-2,2,2-trichloroethyl-oxycarbonyl-L-leucyl-L-norleucinal was obtained in the form of crystals.

### Example 11

### Production of N-trimethylsilylethyloxycarbonyl-L-leucyl-L-norleucinal [in general formula (1), R₁=(CH₃)₃SiCH₂CH₂-OCO-, R₂ = H-, R₃=(CH₃)₂CHCH₂-, R₄ = CH₃(CH₂)₃-]:

### (a) Production of p-nitrophenyl trimethylsilylethyloxycarbonate

4.4 g of trimethylsilylethanol and 10 g of bis(p-nitrophenyl) carbonate were dissolved in 100 ml of dry methylene chloride and 6.0 g of N-methylmorpholine was added thereto. The mixture so formed was stirred at room temperature for 12 hours. After the completion of the reaction, the reaction mixture was successively washed with 0.1% sulfuric acid, a saturated aqueous solution of NaCl, a saturated solution of sodium hydrogencarbonate and a saturated aqueous solution of NaCl and dried over anhydrous sodium sulfate. After distilling off the solvent, the residue was purified by medium pressure column chromatography by use of silica gel. Thus 5.0 g of the titled p-nitrophenyl trimethylsilylethyloxycarbonate was obtained in the form of crystals.

### (b) Production of N-trimethylsilylethyloxycarbonyl-L-leucine dicyclohexylamine salt

4.0 g of L-leucine methyl ester hydrochloride and 2.2 g of triethylamine were dissolved in 100 ml of dry dimethylformamide and 5.8 g of p-nitrophenyl trimethylsilylethyloxycarbonate was as added thereto. To the solution so formed, was added 200 mg of 1-hydroxybenzotriazole monohydride as a catalyst. The resulting mixture was stirred at room temperature for 12 hours. After the completion of the reaction, the reaction mixture was concentrated under reduced pressure and water was added. After extracting with ethyl acetate, the organic phase was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the solvent, the residue was purified by medium pressure column chromatography by use of silica gel. Thus 7.0 g of the N-trimethylsilylethyloxycarbonyl-L-leucine methyl ester was obtained in the form of crystals. The obtained product was dissolved in 20 ml of methanol and 30 ml of a 1 N sodium hydroxide and 50 ml of water were added thereto. The mixture was stirred at 80°C for 1 hour. After the completion of the reaction, the mixture was washed with ethyl acetate and 0.1% of sulfuric acid was added to the aqueous phase to thereby lower the pH value to 2 or below. After extracting with ethyl acetate, the organic phase was dried over anhydrous sodium sulfate and concentrated under reduced pressure. After adding 6.0 g of dicyclohexylamine, 5.5 g of the titled N-trimethylsilylethyloxycarbonyl-L-leucine dicyclohexylamine salt was obtained in the form of crystals.

### (c) Production of N-trimethylsilylethyloxycarbonyl-L-leucyl-L-norleucine methyl ester

4.6 g of the N-trimethylsilylethyloxycarbonyl-L-leucine dicyclohexylamine salt obtained in the above step (b) was suspended in 100 ml of ethyl acetate. After washing with a 10% aqueous solution of citric acid, the organic phase was dried over anhydrous sodium sulfate. The solvent was distilled off under reduced pressure and the N-trimethylsilylethyloxycarbonyl-L-leucine thus obtained was dissolved in 50 ml of dry methylene chloride. Next, 2.0 g of 1-ethyl-3-(3-dimethylaminopropyl)carbodiimide hydrochloride was added thereto. To the solution so formed, was added a solution of 1.8 g of L-norleucine methyl ester hydrochloride and 1.2 g of triethylamine in 50 ml of dry methylene chloride. The mixture was stirred at room temperature for 12 hours. After the completion of the reaction, the reaction mixture was successively washed with 10% citric acid, a saturated aqueous solution of NaCl, a saturated solution of sodium hydrogencarbonate and a saturated aqueous solution of NaCl and dried over anhydrous sodium sulfate. After distilling off the solvent, the residue was purified by medium pressure column chromatography by use of silica gel. Thus 2.8 g of the titled N-trimethylsilylethyloxycarbonyl-L-leucyl-L-norleucine methyl ester was obtained in the form of an oily product.

### (d) Production of N-trimethylsilylethyloxycarbonyl-L-leucyl-L-norleucinol

2.8 g of the N-trimethylsilylethyloxycarbonyl-L-leucyl-L-norleucine methyl ester and 1.5 g of sodium borohydride were suspended in 40 ml of t-butyl alcohol. The resulting mixture was heated under reflux at 90°C under a nitrogen atmosphere. Then 12 ml of absolute methanol was added thereto dropwise under reflux. After the completion of the addition, the reaction mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated solution of NaCl and dried over anhydrous magnesium sulfate. After distilling off the ethyl acetate, the residue was purified by medium pressure column chromatography by use of silica gel. Thus 2.0 g of the titled N-trimethylsilylethyloxycarbonyl-L-leucyl-L-norleucinol was obtained in the form of crystals.

### (e) Production of N-trimethylsilylethyloxycarbonyl-L-leucyl-L-norleucinal

1.8 g of the N-trimethylsilylethyloxycarbonyl-L-leucyl-L-norleucinol obtained in the above step (d) and 1.9 g of triethylamine were dissolved in 20 ml of dry dimethylsulfoxide. To the solution so formed, was added a solution of 3.0 g of sulfur trioxide/pyridine complex dissolved in 20 ml of dimethylsulfoxide. After stirring at room temperature for 20 minutes, the mixture was poured into 300 ml of ice water, thrice extracted with ethyl acetate, successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the obtained residue was purified by reverse phase medium pressure column chromatography by use of octadecyl silane. Thus 1.0 g of the target compound N-trimethylsilylethyloxycarbonyl-L-leucyl-L-norleucinal was obtained in the form of an oily product.

### Example 12

### Production of N-p-toluenesulfonyl-L-leucyl-L-norleucinal [in general formula (1), R₁ = (4-CH₃)C₆H₄SO₂-, R₂ = H-, R₃ = (CH₃)₂CHCH₂-, R₄ = CH₃(CH₂)₃-]:

### (a) Production of N-p-toluenesulfonyl-L-leucine

2.6 ml of L-leucine was dissolved in 40 ml of 2 N sodium hydroxide. 3.8 g of p-toluenesulfonyl chloride was added thereto under ice-cooling and the mixture so formed was stirred at room temperature for 8 hours. After the completion of the reaction, the reaction mixture was washed with ethyl acetate. 5 N hydrochloric acid was added to the aqueous phase to thereby lower the pH value to 2 or below followed by extracting with ethyl acetate. The organic phase was dried over anhydrous sodium sulfate. After distilling off the solvent under reduced pressure, the residue was recrystallized from a solvent mixture of ethyl acetate and hexane. Thus 3.6 g of the titled N-p-toluenesulfonyl-L-leucine was obtained in the form of crystals.

### (b) Production of N-p-toluenesulfonyl-L-leucyl-L-norleucine methyl ester

1.8 g of the N-p-toluenesulfonyl-L-leucine obtained in the above step (a) was dissolved in 50 ml of dry methylene chloride and 1.2 g of 1-ethyl-3-(3-dimethylaminopropyl)carbodiimide hydrochloride was added thereto. To the solution so formed, was added a solution of 1.8 g of L-norleucine methyl ester hydrochloride and 1.2 g of triethylamine dissolved in 50 ml of dry methylene chloride. The resulting mixture was stirred at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 2.8 g of the titled N-p-toluenesulfonyl-L-leucyl-L-norleucine methyl ester was obtained in the form of an oily product.

### (c) Production of N-p-toluenesulfonyl-L-leucyl-L-norleucinol

2.8 g of the N-p-toluenesulfonyl-L-leucyl-L-norleucine methyl ester obtained in the above step (b) and 1.5 g of sodium borohydride were suspended in 40 ml of t-butyl alcohol and then heated under reflux in a nitrogen atmosphere at 90°C. Next, 12 ml of absolute methanol was added thereto dropwise under reflux. After the completion of the addition, the mixture was stirred under reflux for 30 minutes and then cooled to room temperature. 30 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.0 g of the titled N-p-toluenesulfonyl-L-leucyl-L-norleucinol was obtained in the form of crystals.

### (d) Production of N-p-toluenesulfonyl-L-leucyl-L-norleucinal

1.0 g of the N-p-toluenesulfonyl-L-leucyl-L-norleucinol obtained in the above step (c) and 1.2 g of triethylamine were dissolved in 12 ml of dry dimethylsulfoxide. Then a solution of 2.0 g of sulfur trioxide/pyridine complex dissolved in 12 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 0.7 g of the target compound N-p-toluenesulfonyl-L-leucyl-L-norleucinal was obtained in the form of crystals.

### Example 13

### Production of N-o-nitrophenylsulfenyl-L-leucyl-L-norleucinal [in general formula (1), R₁ = (2-NO₂)C₆H₄S-, R₂ = H-, R₃ = (CH₃)₂CHCH₂-, R₄ = CH₃(CH₂)₃-]:

### (a) Production of N-o-nitrophenylsulfenyl-L-leucine dicyclohexylamine salt

5.0 g of L-leucine and 8.0 g of o-nitrophenylsulfenyl chloride were dissolved in 25 ml of a 2 N solution of sodium hydroxide and 25 ml of 1,4-dioxane was added thereto. The mixture so formed was stirred at room temperature for 1 hour. After the completion of the reaction, 2 N sulfuric acid was added to the reaction mixture followed by extracting with ether. The organic phase was dried over sodium sulfate. After distilling of the solvent under reduced pressure, 8.0 g of dicyclohexylamine was added. Thus 10 g of the titled N-o-nitrophenylsulfenyl-L-leucine dicyclohexylamine salt was obtained in the form of crystals.

### (b) Production of N-o-nitrophenylsulfenyl-L-leucyl-L-norleucine methyl ester

4.6 g of the N-o-nitrophenylsulfenyl-L-leucine dicyclohexylamine salt obtained in the above step (a) was suspended in 100 ml of ethyl acetate and washed with a 10% aqueous solution of citric acid. Then the organic phase was dried over sodium sulfate. After distilling off the solvent, the obtained N-o-nitrophenylsulfenyl-L-leucine was dissolved in 50 ml of dry methylene chloride and 2.0 g of 1-ethyl-3-(3-dimethylaminopropyl)carbodiimide hydrochloride was added thereto. To the solution so formed, was added a solution of 1.8 g of L-norleucine methyl ester hydrochloride and 1.0 g of triethylamine dissolved in 50 ml of dry methylene chloride. The resulting mixture was stirred at room temperature for 12 hours. After the completion of the reaction, the reaction mixture was successively washed with a 10% citric acid, a saturated aqueous solution of NaCl, a saturated solution of sodium hydrogencarbonate and a saturated aqueous solution of NaCl and then dried over anhydrous sodium sulfate. After distilling off the solvent under reduced pressure, the residue was purified by medium pressure column chromatography by use of silica gel. Thus 5.0 g of the titled N-o-nitrophenylsulfenyl-L-leucyl-L-norleucine methyl ester was obtained in the form of an oily product.

### (c) Production of N-o-nitrophenylsulfenyl-L-leucyl-L-norleucinol

3.2 g of the N-o-nitrophenylsulfenyl-L-leucyl-L-norleucine methyl ester obtained in the above step (b) and 2.0 g of sodium borohydride were suspended in 50 ml of t-butyl alcohol and then heated under reflux in a nitrogen atmosphere at 90°C. Next, 15 ml of absolute methanol was added thereto dropwise under reflux. After the completion of the addition, the mixture was stirred under reflux for 30 minutes and then cooled to room temperature. 50 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.0 g of the titled N-o-nitrophenylsulfenyl-L-leucyl-L-norleucinol was obtained in the form of crystals.

### (d) Production of N-o-nitrophenylsulfenyl-L-leucyl-L-norleucinal

1.0 g of the N-o-nitrophenylsulfenyl-L-leucyl-L-norleucinol obtained in the above step (c) and 1.2 g of triethylamine were dissolved in 12 ml of dry dimethylsulfoxide. Then a solution of 2.0 g of sulfur trioxide/pyridine complex dissolved in 12 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was purified by reverse phase medium pressure column chromatography by use of octadecyl silane. Thus 1.2 g of the target compound N-o-nitrophenylsulfenyl-L-leucyl-L-norleucinal was obtained in the form of an oily product.

### Example 14

### Production of N-diphenylphosphonothioyl-L-leucyl-L-norleucinal [in general formula (1), R₁ = (C₆H₅)₂PS-, R₂ = H-, R₃ = (CH₃)₂CHCH₂-, R₄ = CH₃(CH₂)₃-]:

### (a) Production of N-diphenylphosphonothioyl-L-leucyl-L-norleucine methyl ester

2.0 g of N-diphenylphosphonothioyl-L-leucine was dissolved in 50 ml of dry methylene chloride and 2.0 g of 1-ethyl-3-(3-dimethylaminopropyl)carbodiimide hydrochloride was added thereto. To the solution so formed, was added a solution of 1.8 g of L-norleucine methyl ester hydrochloride and 1.0 g of triethylamine dissolved in 50 ml of dry methylene chloride. The resulting mixture was stirred at room temperature for 12 hours. After the completion of the reaction, the reaction mixture was successively washed with 10% citric acid, a saturated aqueous solution of NaCl, a saturated solution of sodium hydrogencarbonate and a saturated aqueous solution of NaCl and dried over anhydrous sodium sulfate. After distilling off the solvent, the residue was purified by medium pressure column chromatography by use of silica gel. Thus 3.0 g of the titled N-diphenylphosphonothioyl-L-leucyl-L-norleucine methyl ester was obtained in the form of an oily product.

### (b) Production of N-diphenylphosphonothioyl-L-leucyl-L-norleucinol

2.5 g of the N-diphenylphosphonothioyl-L-leucyl-L-norleucine methyl ester obtained in the above step (a) and 1.0 g of sodium borohydride were suspended in 50 ml of t-butyl alcohol and then heated under reflux in a nitrogen atmosphere at 90°C. Next, 10 ml of absolute methanol was added thereto dropwise under reflux. After the completion of the addition, the mixture was stirred under reflux for 30 minutes and then cooled to room temperature. 50 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.0 g of the titled N-diphenylphosphonothioyl-L-leucyl-L-norleucinol was obtained in the form of crystals.

### (c) Production of N-diphenylphosphonothioyl-L-leucyl-L-norleucinal

1.0 g of of the N-diphenylphosphonothioyl-L-leucyl-L-norleucinol obtained in the above step (b) and 0.8 g of triethylamine were dissolved in 8 ml of dry dimethylsulfoxide. To the solution so formed, was added a solution of 1.3 g of sulfur trioxide/pyridine complex in 8 ml of dimethylsulfoxide. The mixture was stirred at room temperature for 10 minutes and then poured into 200 ml of ice-water. After thrice extracting with ethyl acetate, the extract was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was purified by reverse phase medium pressure column chromatography by use of octadecyl silane. Thus, 0.6 g of the target compound N-diphenylphosphonothioyl-L-leucyl-L-norleucinal was obtained in the form of an oily product.

### Example 15

### Production of N-triphenylmethyl-L-leucyl-L-norleucinal [in general formula (1), R₁ = (C₆H₅)₃C-, R₂ = H-, R₃ = (CH₃)₂CHCH₂-, R₄ = CH₃(CH₂)₃-]:

### (a) Production of N-benzyloxycarbonyl-L-leucyl-norleucine methyl ester

7.4 g of N-benzyloxycarbonyl-L-leucine was dissolved in 100 ml of dry methylene chloride and 8.2 g of 1-ethyl-3-(3-dimethylaminopropyl)carbodiimide hydrochloride was added thereto. To the solution so formed, was added a solution of 7.4 g of L-norleucine methyl ester hydrochloride and 4.2 g of triethylamine dissolved in 100 ml of dry methylene chloride. The resulting mixture was stirred at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and dried over anhydrous sodium sulfate. After distilling off the solvent, the residue was purified by medium pressure column chromatography by use of silica gel. Thus 12 g of the titled N-benzyloxycarbonyl-L-leucyl-L-norleucine methyl ester was obtained in the form of crystals.

### (b) Production of N-triphenylmethyl-L-leucyl-L-norleucine methyl ester

4.0 g of the N-benzyloxycarbonyl-L-leucyl-L-norleucine methyl ester obtained in the above step (a) was dissolved in 50 ml of ethanol. Then a catalytic amount of palladium carbon was added thereto and the mixture so formed was stirred under a hydrogen atmosphere for 3 hours. After the completion of the reaction, the palladium carbon was filtered off and the solvent was distilled off from the filtrate under reduced pressure. Thus L-leucyl-L-norleucine methyl ester was quantitatively obtained. Next, this product was dissolved in 100 ml of dry methylene chloride and 1.5 g of triethylamine was added thereto. Further, 4.2 g of triphenylmethane chloride was added and the resulting mixture was stirred at room temperature for 12 hours. After the completion of the reaction, the reaction mixture was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the solvent under reduced pressure, the residue was purified by medium pressure column chromatography by use of silica gel. Thus 4.3 g of the tiled N-triphenylmethyl-L-leucyl-L-norleucine methyl ester was obtained in the form of crystals.

### (c) Production of N-triphenylmethyl-L-leucyl-L-norleucinol

4.3 g of the N-triphenylmethyl-L-leucyl-L-norleucine methyl ester obtained in the above step (b) and 1.5 g of sodium borohydride were suspended in 50 ml of t-butyl alcohol and then heated under reflux in a nitrogen atmosphere at 90°C. Next, 15 ml of absolute methanol was added thereto dropwise under reflux. After the completion of the addition, the mixture was stirred under reflux for 30 minutes and then cooled to room temperature. 100 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.8 g of the titled N-triphenylmethyl-L-leucyl-L-norleucinol was obtained in the form of powdery crystals.

### (d) Production of N-triphenylmethyl-L-leucyl-L-norleucinal

1.0 g of the N-triphenylmethyl-L-leucyl-L-norleucinol obtained in the above step (c) and 2.1 g of triethylamine were dissolved in 10 ml of dry dimethylsulfoxide. To the solution so formed, was added a solution of 2.0 g of sulfur trioxide/pyridine complex dissolved in 10 ml of dimethylsulfoxide. The mixture was stirred at room temperature for 10 minutes and then poured into 200 ml of ice-water. After thrice extracting with ethyl acetate, the extract was successively washed with a 10% aqueous solution of citric acid, a saturated aqueous solution of NaCl, a saturated solution of sodium hydrogencarbonate and a saturated aqueous solution of NaCl and dried over anhydrous sodium sulfate.

After distilling off the ethyl acetate, the obtained residue was recrystallized from a solvent mixture of ethyl acetate and hexane. Thus 0.7 g of the target compound N-triphenylmethyl-L-leucyl-L-norleucinal was obtained in the form of crystals.

### Example 16

### Production of N-(2-benzoyl-1-methyl)vinyl-L-leucyl-L-norleucinal [in general formula (1), R₁ = C₆H₅COCH=C(CH₃)-, R₂ = H-, R₃ = (CH₃)₂CHCH₂-, R₄ = CH₃(CH₂)₃-]:

### (a) Production of N-(2-benzoyl-1-methyl)vinyl-L-leucine

6.6 g of benzoylacetone was dissolved in 100 ml of dry ethanol. To the solution so formed, was added a solution of 1.8 g of sodium hydroxide dissolved in 20 ml of dry methanol. 5.4 g of L-leucine was further added thereto and the resulting mixture was heated under reflux at 100°C for 3 hours. After the completion of the reaction, the solvent was concentrated under reduced pressure and water was added. The pH value of the reaction mixture was lowered to around 2 with 10% citric acid. Then the mixture was extracted with ether and dried over anhydrous sodium sulfate followed by distilling off the solvent under reduced pressure. The residue was recrystallized from a solvent mixture of ethyl acetate and hexane. Thus 7.0 g of the titled N-(2-benzoyl-1-methyl)vinyl-L-leucine was obtained in the form of crystals.

### (b) Production of N-(2-benzoyl-1-methyl)vinyl-L-leucyl-L-norleucine methyl ester

2.8 g of the N-(2-benzoyl-1-methyl)vinyl-L-leucine obtained in the above step (a) was dissolved in 50 ml of dry methylene chloride and 2.0 g of 1-ethyl-3-(3-dimethylaminopropyl)carbodiimide hydrochloride was added thereto. To the solution so formed, was added a solution of 1.8 g of L-norleucine methyl ester hydrochloride and 1.0 g of triethylamine dissolved in 50 ml of dry methylene chloride. The mixture was stirred at room temperature for 12 hours. After the completion of the reaction, the reaction mixture was successively washed with 10% citric acid, a saturated aqueous solution of NaCl, a saturated solution of sodium hydrogencarbonate and a saturated aqueous solution of NaCl and then dried over sodium sulfate. After distilling off the solvent under reduced pressure, the residue was purified by medium pressure column chromatography by use of silica gel. Thus 3.5 g of the titled N-(2-benzoyl-1-methyl)vinyl-L-leucyl-L-norleucine methyl ester was obtained in the form of an oily product.

### (c) Production of N-(2-benzoyl-1-methyl)vinyl-L-leucyl-L-norleucinol

2.5 g of the N-(2-benzoyl-1-methyl)vinyl-L-leucyl-L-norleucine methyl ester obtained in the above step (b) and 1.0 g of sodium borohydride were suspended in 50 ml of t-butyl alcohol and then heated under reflux in a nitrogen atmosphere at 90°C. Next, 10 ml of absolute methanol was added thereto dropwise under reflux. After the completion of the addition, the mixture was stirred under reflux for 30 minutes and then cooled to room temperature. 50 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.0 g of the titled N-(2-benzoyl-1-methyl)vinyl-L-leucyl-L-norleucinol was obtained in the form of crystals.

### (d) Production of N-(2-benzoyl-1-methyl)vinyl-L-leucyl-L-norleucinal

1.0 g of the N-(2-benzoyl-1-methyl)vinyl-L-leucyl-L-norleucinol obtained in the above step (c) and 1.2 g of triethylamine were dissolved in 12 ml of dry dimethylsulfoxide. Then a solution of 2.0 g of sulfur trioxide/pyridine complex dissolved in 12 ml of dimethylsulfoxide was added thereto under stirring. The mixture was stirred at room temperature for 10 minutes and then poured into 200 ml of ice water. After thrice extracting with ethyl acetate, it was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was purified by reverse phase medium pressure column chromatography by use of octadecyl silane. Thus 0.6 g of the target compound N-(2-benzoyl-1-methyl)vinyl-L-leucyl-L-norleucinal was obtained in the form of an oily product.

### Example 17

### Production of N-phthaloyl-L-leucyl-L-norleucinal [in general formula (1), R₁ and R₂ form a 1,2-(CO-)₂C₆H₄ group together, R₃ = (CH₃)₂CHCH₂-, R₄ = CH₃(CH₂)₃-]:

### (a) Production of N-benzyloxycarbonyl-L-leucyl-L-norleucine methyl ester

7.4 g of N-benzyloxycarbonyl-L-leucine was dissolved in 100 ml of dry methylene chloride and 8.2 g of 1-ethyl-3-(3-dimethylaminopropyl)carbodiimide hydrochloride was added thereto. To the solution so formed, was added a solution of 7.4 g of L-norleucine methyl ester hydrochloride and 4.2 g of triethylamine dissolved in 100 ml of dry methylene chloride. The resulting mixture was stirred at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and dried over anhydrous sodium sulfate. After distilling off the solvent, the residue was purified by medium pressure column chromatography by use of silica gel. Thus 12 g of the titled N-benzyloxycarbonyl-L-leucyl-L-norleucine methyl ester was obtained in the form of crystals.

### (b) Production of N-benzyloxycarbonyl-L-leucyl-L-norleucinol

10 g of the N-benzyloxycarbonyl-L-leucyl-L-norleucine methyl ester obtained in the above step (a) and 3.0 g of sodium borohydride were suspended in 100 ml of t-butyl alcohol and then heated under reflux in a nitrogen atmosphere at 90°C. Next, 20 ml of absolute methanol was added thereto dropwise under reflux. After the completion of the addition, the mixture was stirred under reflux for minutes and then cooled to room temperature. 100 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 8.0 g of the titled N-benzyloxycarbonyl-L-leucyl-L-norleucinol was obtained in the form of crystals.

### (c) Production of N-phthaloyl-L-leucyl-L-norleucinol

2.5 g of the N-benzyloxycarbonyl-L-leucyl-L-norleucinol obtained in the above step (b) was dissolved in 50 ml of ethanol and a catalytic amount of palladium carbon was added thereto. The mixture was stirred under a hydrogen atmosphere for 3 hours. After the completion of the reaction, the palladium carbon was filtered off and the solvent was distilled off from the filtrate. Thus L-leucyl-L-norleucinol was quantitatively obtained. To the product so formed, were added 20 ml of water, 2.2 g of potassium carbonate and 1.6 g of carboethoxyphthalimide. The resulting mixture was stirred at room temperature for 2 hours. After the completion of the reaction, the mixture was thrice extracted with ethyl acetate, washed with a saturated aqueous solution of NaCl and dried over anhydrous magnesium sulfate. After distilling off the ethyl acetate, the obtained residue was purified by medium pressure column chromatography by use of silica gel. Thus 0.5 g of the titled N-phthaloyl-L-leucyl-L-norleucinol was obtained in the form of crystals.

### (d) Production of N-phthaloyl-L-leucyl-L-norleucinal

0.5 g of the N-phthaloyl-L-leucyl-L-norleucinol obtained in the above step (c) and 0.7 g of triethylamine were dissolved in 6 ml of dry dimethylsulfoxide. To the solution so formed, was added a solution of 1.0 g of sulfur trioxide/pyridine complex dissolved in 6 ml of dimethylsulfoxide under stirring. The resulting mixture was stirred at room temperature for 10 minutes and poured into 100 ml of ice water. Then it was thrice extracted with ethyl acetate, successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the solvent, the residue was recrystallized from a solvent mixture of ethyl acetate and hexane. Thus 0.3 g of the target compound N-phthaloyl-L-leucyl-L-norleucinal was obtained in the form of crystals.

### Example 18

### Production of N-benzyloxycarbonyl-L-norleucyl-L-norleucinal [in general formula (1), R₁ = C₆H₅CH₂OCO-, R₂ = H-, R₃ = CH₃(CH₂)₃-, R₄ = CH₃(CH₂)₃-]:

### (a) Production of N-benzyloxycarbonyl-L-norleucine

7.8 g of L-norleucine and 8.4 g of potassium carbonate were dissolved in 200 ml of water. Then a solution of 12.4 g of benzyloxycarbonyl chloride dissolved in 20 ml of dioxane was added thereto under ice-cooling. The resulting mixture was stirred at room temperature for 8 hours. After the completion of the reaction, the reaction mixture was washed with ether and 5 N hydrochloric acid was added to the aqueous phase to thereby lower the pH value to 2 or below. After extracting with ethyl acetate, the organic phase was dried over anhydrous sodium sulfate and the solvent was distilled off. Thus 9.5 g of the titled N-benzyloxycarbonyl-L-norleucine was obtained in the form of an oily product.

### (b) Production of N-benzyloxycarbonyl-L-norleucyl-L-norleucine methyl ester

7.0 g of N-benzyloxycarbonyl-L-norleucine obtained in the above step (a) was dissolved in 100 ml of dry methylene chloride and 5.4 g of 1-ethyl-3-(3-dimethylaminopropyl)carbodiimide hydrochloride was added thereto. To the solution so formed, was added a solution of 4.2 g of L-norleucine methyl ester hydrochloride and 5.2 g of triethylamine in 100 ml of dry methylene chloride. The resulting mixture was stirred at room temperature for 12 hours. After the completion of the reaction, the reaction mixture was successively washed with 1 N hydrochloric acid, a saturated aqueous solution of NaCl, a saturated solution of sodium hydrogencarbonate and a saturated aqueous solution of NaCl and dried over anhydrous sodium sulfate. After distilling off the solvent, the residue was purified by medium pressure column chromatography by use of silica gel. Thus 8.0 g of the titled N-benzyloxycarbonyl-L-norleucyl-L-norleucine methyl ester was obtained in the form of crystals.

### (c) Production of N-benzyloxycarbonyl-L-norleucyl-L-norleucinol

4.0 g of the N-benzyloxycarbonyl-L-norleucyl-L-norleucine methyl ester obtained in the above step (b) and 1.2 g of sodium borohydride were suspended in 100 ml of t-butyl alcohol and then heated under reflux in a nitrogen atmosphere at 90°C. Next, 16 ml of absolute methanol was added thereto dropwise under reflux. After the completion of the addition, the mixture was stirred under reflux for 30 minutes and then cooled to room temperature. 50 ml of water was added thereto under ice-cooling. After distilling off the methanol and t-butyl alcohol under reduced pressure, the residue was thrice extracted with ethyl acetate, washed with a saturated aqueous solution of NaCl and dried over anhydrous magnesium sulfate. The ethyl acetate was distilled off under reduced pressure and the obtained residue was purified by medium pressure column chromatography by use of silica gel. Thus 2.2 g of the titled N-benzyloxycarbonyl-L-norleucyl-L-norleucinol was obtained in the form of crystals.

### (d) Production of N-benzyloxycarbonyl-L-norleucyl-L-norleucinal

1.7 g of the N-benzyloxycarbonyl-L-norleucyl-L-norleucinol obtained in the above step (c) and 1.8 g of triethylamine were dissolved in 12 ml of dry dimethylsulfoxide. To the solution so formed, was added a solution of 3.0 g of sulfur trioxide/pyridine complex dissolved in 12 ml of dimethylsulfoxide under stirring. The mixture was stirred at room temperature for 10 minutes and then poured into 200 ml of ice water. After thrice extracting with ethyl acetate, the extract was successively washed with a 10% aqueous solution of citric acid, a saturated aqueous solution of NaCl, a saturated solution of sodium hydrogencarbonate and a saturated aqueous solution of NaCl and dried over anhydrous sodium sulfate. After distilling off the ethyl acetate, the residue was recrystallized from a solvent mixture of ethyl acetate and hexane. Thus 1.2 g of the target compound N-benzyloxycarbonyl-L-norleucyl-L-norleucinal was obtained in the form of crystals.

Table 1 shows the physical properties of each compound.

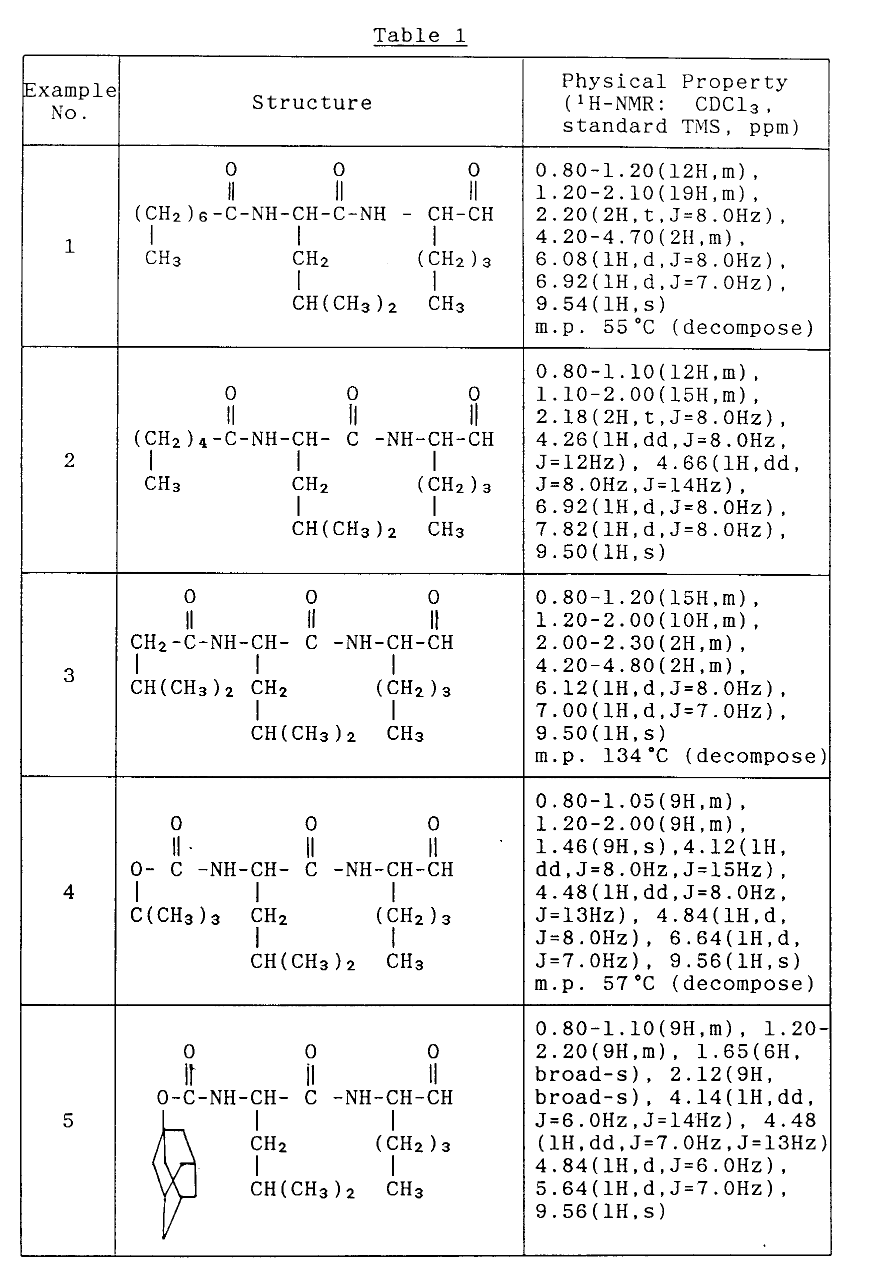

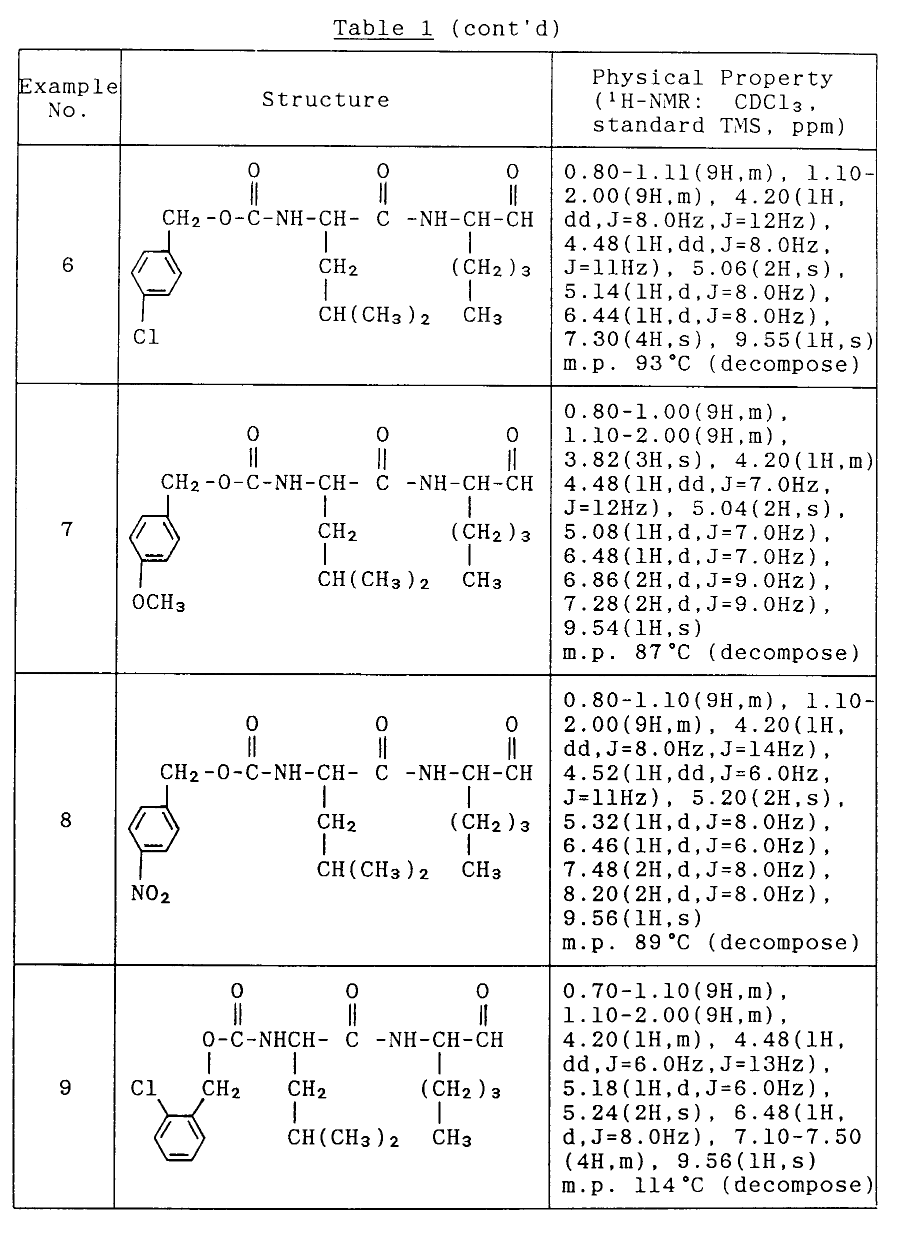

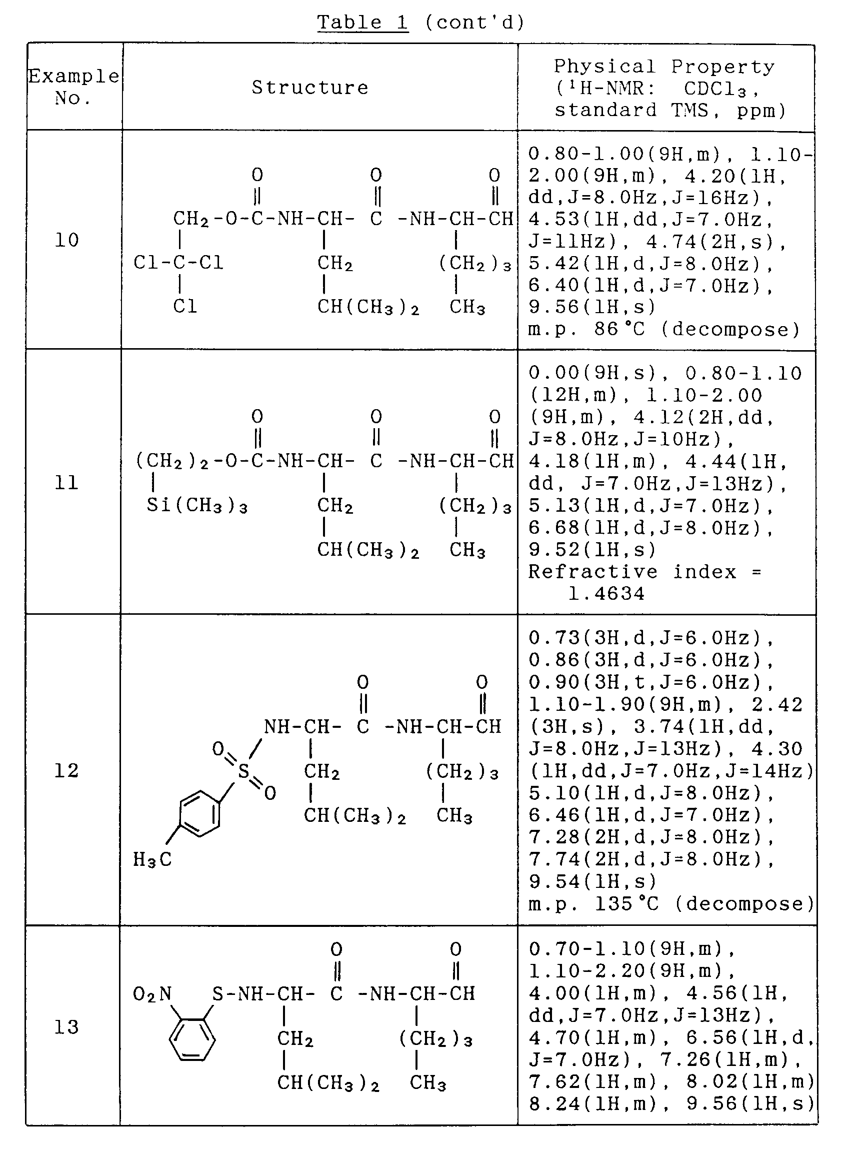

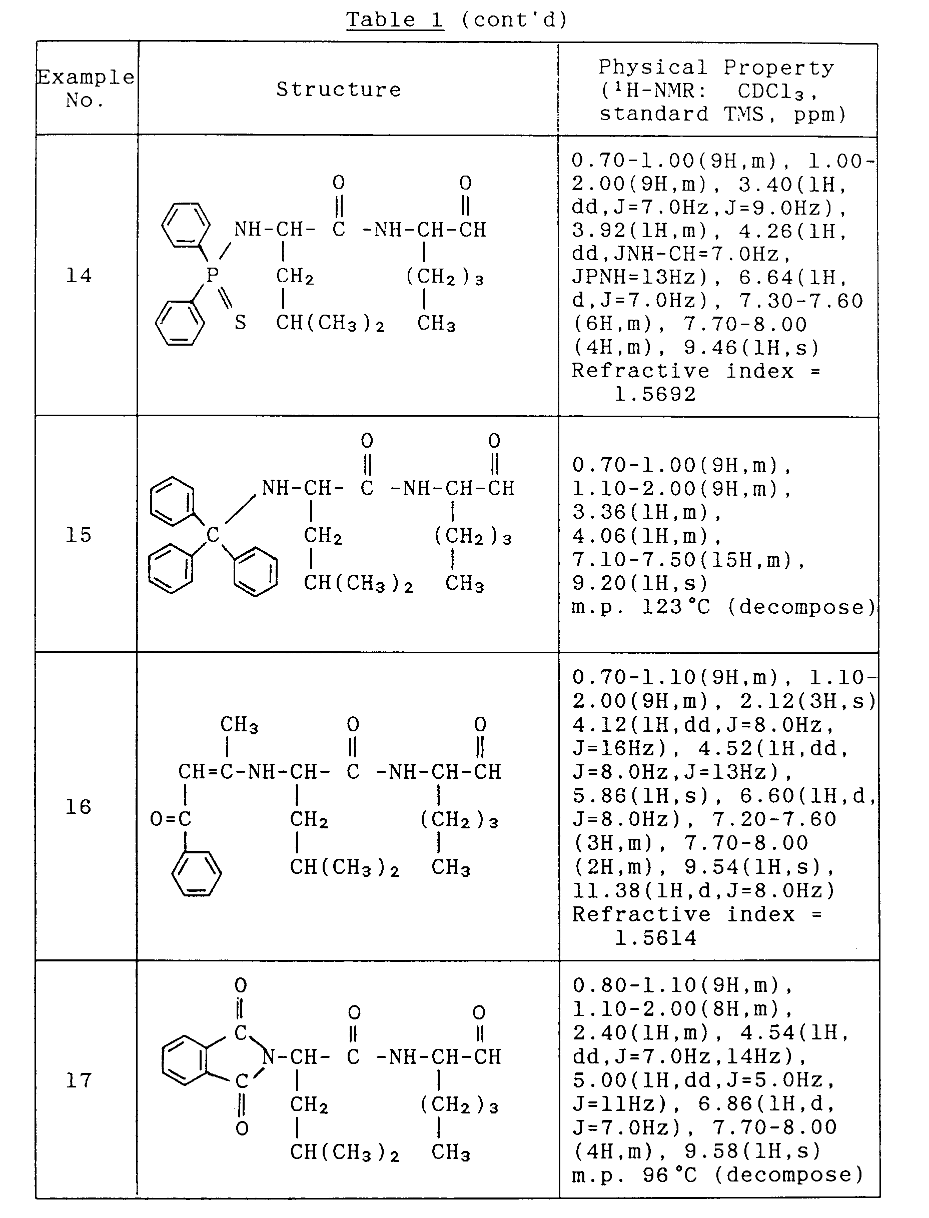

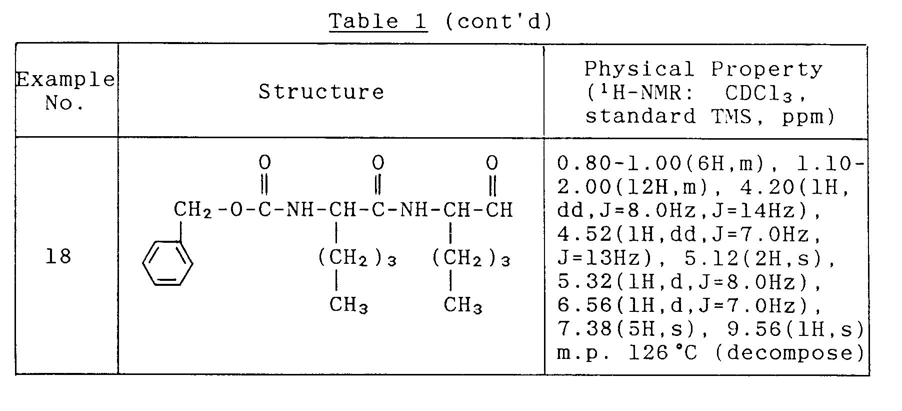

### Example 19

### Enzyme Inhibitory Activity of the Invention Compound

The enzyme inhibitory activities of the compounds of the present invention were evaluated in the following manner.

The antipapain activity was determined as follows. A compound of the present invention at various concentrations, 0.015 U of papain and 0.88 mg of EGTA were dissolved in 1 ml of a citrate buffer solution (20 mM, pH = 6.2). The solution so formed was preincubated at 30°C for 5 minutes. Then 1 ml of a substrate solution was added to initiate the reaction. As a substrate, a 1% solution of casein in a citrate buffer solution was employed. This reaction was conducted at 30°C for 20 minutes. Next, 3 ml of 6.5% trichloroacetic acid was added to the reaction mixture to thereby stop the reaction. The amount of proteins in the trichloroacetic acid-soluble fraction of the casein digested with the enzyme was determined by Lowry-Folin method. The obtained data were compared with control data so as to determine the inhibitory activity.

The anticalpain activity, i.e., the anticalpain I or anticalpain II activity was determined as follows. A compound of the present invention at various concentrations, 0.33 U of calpain I or II and 0.22 mg of calcium chloride were dissolved in 1 ml of an imidazole/hydrochloride buffer solution (50 mM, pH = 7.5). The solution so formed was preincubated at 30°C for 5 minutes. Then 1 ml of a substrate solution was added to initiate the reaction. As a substrate, a 0.4% solution of casein in an imidazole/hydrochloride buffer solution was employed. This reaction was conducted at 30°C for 30 minutes. Next, 3 ml of 5% trichloroacetic acid was added to stop the reaction. The amount of proteins in the trichloroacetic acid-soluble fraction of the casein digested with the enzyme was determined by Ross-Scahtz method. The obtained data were compared with control data so as to determine the inhibitory activity.

The anticathepsin activity was determined as follows. A solution comprising a compound of the present invention at various concentrations and 0.114 mg of a substrate N-benzyloxycarbonyl-L-lysine p-nitrophenyl ester) in 3.15 ml of an acetate buffer solution (25 mM, pH = 5.1, containing 1 mM of EDTA) was preincubated at 30°C for 1 minute. Then a solution of 0.05 U of cathepsin B originating from bovine spleen (Sigma Co.) dissolved in the same buffer (0.05 ml) was added to initiate the reaction. A change in the absorbance at 326 nm was monitored immediately after the initiation of the reaction. The enzyme inhibitory activity was determined by comparing the data with those obtained by use of a control solution.

Tables 2, 3, 4 and 5 show the inhibitory activities of the compounds of the present invention respectively on papain, calpains and cathepsin B, which is the target enzyme, thus determined. In the cases of papain and calpains, calpeptin (Tsujinaka et al., Biochem. Biophys. Res. Commune., 153, 1201 - 1208, 1988) was employed as a control.

**Table 2**

| Inhibitory Activity on Papain | |
|---|---|
| Example No. | Inhibitory activity IC₅₀ (M) |
| 3 | 1.2 x 10⁻⁷ |
| 5 | 2.1 x 10⁻⁷ |
| 6 | 5.8 x 10⁻⁸ |
| 7 | 2.5 x 10⁻⁷ |
| 8 | 3.1 x 10⁻⁸ |
| 9 | 1.6 x 10⁻⁷ |
| 10 | 2.6 x 10⁻⁷ |
| 12 | 1.8 x 10⁻⁷ |
| 14 | 1.7 x 10⁻⁷ |
| 16 | 9.6 x 10⁻⁸ |
| Calpeptin | 3.1 x 10⁻⁷ |

**Table 3**

| Inhibitory Activity on Calpain I | |
|---|---|
| Example No. | Inhibitory activity IC₅₀ (M) |
| 1 | 1.6 x 10⁻⁷ |
| 3 | 1.0 x 10⁻⁷ |
| 4 | 3.0 x 10⁻⁸ |
| 5 | 1.5 x 10⁻⁷ |
| 6 | 8.7 x 10⁻⁸ |
| 7 | 1.0 x 10⁻⁷ |
| 8 | 9.2 x 10⁻⁸ |
| 9 | 1.1 x 10⁻⁷ |
| 10 | 9.6 x 10⁻⁸ |
| 12 | 1.1 x 10⁻⁷ |
| 14 | 9.0 x 10⁻⁸ |
| 16 | 4.0 x 10⁻⁷ |
| 18 | 7.0 x 10⁻⁷ |
| Calpeptin | 7.1 x 10⁻⁷ |

**Table 4**

| Inhibitory Activity on Calpain II | |
|---|---|
| Example No. | Inhibitory activity IC₅₀ (M) |
| 3 | 1.3 x 10⁻⁷ |
| 4 | 1.2 x 10⁻⁷ |
| 6 | 1.3 x 10⁻⁷ |
| 7 | 1.3 x 10⁻⁷ |
| 8 | 1.3 x 10⁻⁷ |
| 9 | 1.3 x 10⁻⁷ |
| 14 | 1.2 x 10⁻⁷ |
| Calpeptin | 1.3 x 10⁻⁷ |

**Table 5**

| Inhibitory Activity on Cathepsin B | |
|---|---|
| Example No. | Inhibitory activity IC₅₀ (M) |
| 1 | 1.1 x 10⁻⁷ |
| 2 | 9.2 x 10⁻⁶ |
| 3 | 5.1 x 10⁻⁷ |
| 4 | 5.8 x 10⁻⁸ |
| 5 | 1.8 x 10⁻⁷ |
| 6 | 7.0 x 10⁻⁸ |
| 7 | 1.5 x 10⁻⁷ |
| 8 | 9.9 x 10⁻⁵ |
| 9 | 1.4 x 10⁻⁷ |
| 10 | 3.3 x 10⁻⁷ |
| 11 | 9.6 x 10⁻⁷ |
| 12 | 1.2 x 10⁻⁶ |
| 14 | 7.9 x 10⁻⁸ |
| 15 | 3.2 x 10⁻⁵ |
| 16 | 7.0 x 10⁻⁶ |
| 17 | 1.0 x 10⁻⁴ |
| 18 | 1.1 x 10⁻⁷ |

## Claims (Claims for the following Contracting State(s): AT, BE, CH, DE, DK, FR GB, IT, LI, LU, NL, SE)

1. A compound represented by the following general formula (1): 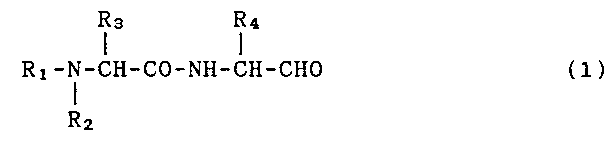 wherein R₁ represents a straight-chain or branched acyl group having 2 to 10 carbon atoms, a branched-, cyclic- or polycyclic-alkyloxycarbonyl group having 4 to 15 carbon atoms, a substituted- or unsubstituted-benzyloxycarbonyl group, a 2,2,2-trichloroethyloxycarbonyl group, a 2-(trimethylsilyl)ethyloxycarbonyl group, a p-toluenesulfonyl group, an o-nitrophenylsulfenyl group, a diphenylphosphonothioyl group, a triphenylmethyl group or a 2-benzoyl-1-methylvinyl group;
R₂ represents a hydrogen atom; or R₁ and R₂ may together form a phthaloyl group;
R₃ represents an isobutyl group or a n-butyl group and the above-mentioned R₁ can be an unsubstituted-benzyloxycarbonyl group provided that R₃ is a n-butyl group; and
R₄ represents a n-butyl group.

2. A compound as claimed in Claim 1, wherein R₁ is a straight-chain or branched acyl group having 2 to 10 carbon atoms selected from among octanoyl, caproyl and isovaleryl groups.

3. A compound as claimed in Claim 1, wherein R₁ is a branched-, cyclic- or polycyclic-alkyloxycarbonyl group having 4 to 15 carbon atoms selected from among t-butyloxycarbonyl, adamantyloxycarbonyl and isobornyloxycarbonyl groups.

4. A compound as claimed in Claim 1, wherein R₁ is a substituted-benzyloxycarbonyl group having one or more substituents selected from among halogen atoms, nitro group and methoxy group.

5. A proteinase inhibitor which contains a compound represented by the general formula (1) shown in Claim 1 as an active ingredient.

6. A pharmaceutical composition, comprising a compound as defined in claim 1 as an active ingredient and one or more pharmaceutically acceptable carriers.

7. Use of a compound as defined in claim 1 for making a medicament, effective in inhibiting proteinases.

## Claims (Claims for the following Contracting State(s): ES)

1. Process for preparing a compound having the general formula (1): 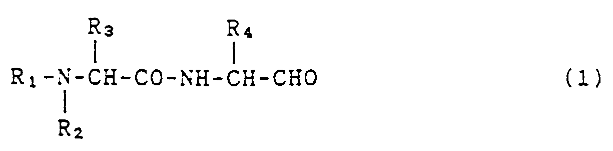 wherein R₁ represents a straight-chain or branched acyl group having 2 to 10 carbon atoms, a branched-, cyclic- or polycyclic-alkyloxycarbonyl group having 4 to 15 carbon atoms, a substituted- or unsubstituted-benzyloxycarbonyl group, a 2,2,2-trichloroethyloxycarbonyl group, a 2-(trimethylsilyl)ethyloxycarbonyl group, a p-toluenesulfonyl group, an o-nitrophenylsulfenyl group, a diphenylphosphonothioyl group, a triphenylmethyl group or a 2-benzoyl-1-methylvinyl group;
R₂ represents a hydrogen atom; or R, and R₂ may together form a phthaloyl group;
R₃ represents an isobutyl group or a n-butyl group and the above-mentioned R₁ can be an unsubstituted-benzyloxycarbonyl group provided that R₃ is a n-butyl group; and
R₄ represents a n-butyl group,
comprising the following steps:
reducing a compound represented by the following general formula (II): 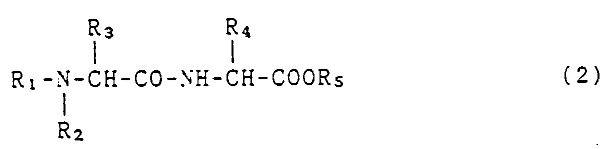 wherein R₁, R₂, R₃ and R₄ are as defined above; and
wherein R₅ represents a lower alkyl group,
by use of a reducing agent in an organic solvent to obtain an alcohol compound;
or reducing a compound represented by the following general formula (III): 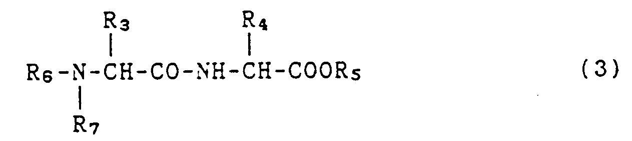 wherein R₃, R₄ and R₅ are as defined above regarding the above general formula (2);
R₆ represents an amino-protecting group which is stable to a treatment with a base or reduction by use of a reducing agent; and
R₇ represents a hydrogen atom; or
R₆ and R₇ together form an amino-protecting group which is stable to a treatment with a base or reduction by use of a reducing agent;
by use of a reducing agent in an organic solvent to give an alcohol compound,
oxidizing said alcohol compound by use of an oxidizing agent to give the aldehyde.

2. A process as claimed in Claim 1, wherein R₁ is a straight-chain or branched acyl group having 2 to 10 carbon atoms selected from among octanoyl, caproyl and isovaleryl groups.

3. A process as claimed in Claim 1, wherein R₁ is a branched-, cyclic- or polycyclic-alkyloxycarbonyl group having 4 to 15 carbon atoms selected from among t-butyloxycarbonyl, adamantyloxycarbonyl and isobornyloxycarbonyl groups.

4. A process as claimed in Claim 1, wherein R₁ is a substituted-benzyloxycarbonyl group having one or more substituents selected from among halogen atoms, nitro group and methoxy group.

5. A process for preparing a pharmaceutical composition by combining a compound as defined in claim 1 as an active ingredient with one or more pharmaceutically acceptable carriers.

6. A process for preparing a pharmaceutical composition as claimed in claim 5 for use as an inhibitor of proteinases.

## Claims (Claims for the following Contracting State(s): GR)

1. Process for preparing a compound having the general formula (1): 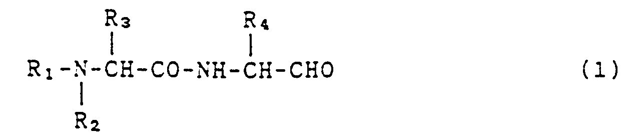 wherein R₁ represents a straight-chain or branched acyl group having 2 to 10 carbon atoms, a branched-, cyclic- or polycyclic-alkyloxycarbonyl group having 4 to 15 carbon atoms, a substituted- or unsubstituted-benzyloxycarbonyl group, a 2,2,2-trichloroethyloxycarbonyl group, a 2-(trimethylsilyl)ethyloxycarbonyl group, a p-toluenesulfonyl group, an o-nitrophenylsulfenyl group, a diphenylphosphonothioyl group, a triphenylmethyl group or a 2-benzoyl-1-methylvinyl group;
R₂ represents a hydrogen atom; or R₁ and R₂ may together form a phthaloyl group;
R₃ represents an isobutyl group or a n-butyl group and the above-mentioned R₁ can be an unsubstituted-benzyloxycarbonyl group provided that R₃ is a n-butyl group; and
R₄ represents a n-butyl group,
comprising the following steps:
reducing a compound represented by the following general formula (II): 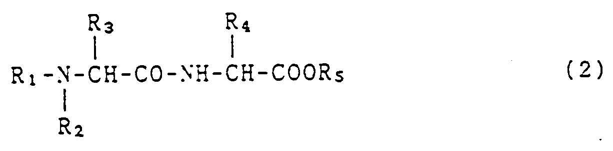 wherein R₁, R₂, R₃ and R₄ are as defined above; and
wherein R₅ represents a lower alkyl group,
by use of a reducing agent in an organic solvent to obtain an alcohol compound;
or reducing a compound represented by the following general formula (III): 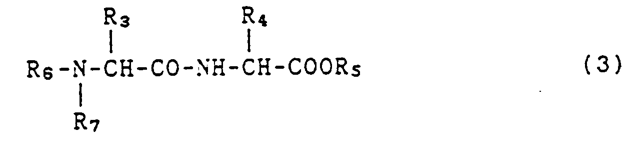 wherein R₃, R₄ and R₅ are as defined above regarding the above general formula (2);
R₆ represents an amino-protecting group which is stable to a treatment with a base or reduction by use of a reducing agent; and
R₇ represents a hydrogen atom; or
R₆ and R₇ together form an amino-protecting group which is stable to a treatment with a base or reduction by use of a reducing agent;
by use of a reducing agent in an organic solvent to give an alcohol compound,
oxidizing said alcohol compound by use of an oxidizing agent to give the aldehyde.

2. A compound represented by the following general formula (1): 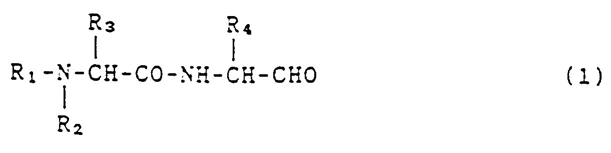 wherein R₁ represents a straight-chain or branched acyl group having 2 to 10 carbon atoms, a branched-, cyclic- or polycyclic-alkyloxycarbonyl group having 4 to 15 carbon atoms, a substituted- or unsubstituted-benzyloxycarbonyl group, a 2,2,2-trichloroethyloxycarbonyl group, a 2-(trimethylsilyl)ethyloxycarbonyl group, a p-toluenesulfonyl group, an o-nitrophenylsulfenyl group, a diphenylphosphonothioyl group, a triphenylmethyl group or a 2-benzoyl-1-methylvinyl group;
R₂ represents a hydrogen atom; or R₁ and R₂ may together form a phthaloyl group;
R₃ represents an isobutyl group or a n-butyl group and the above-mentioned R₁ can be an unsubstituted-benzyloxycarbonyl group provided that R₃ is a n-butyl group; and
R₄ represents a n-butyl group.

3. A compound as claimed in Claim 2, wherein R₁ is a straight-chain or branched acyl group having 2 to 10 carbon atoms selected from among octanoyl, caproyl and isovaleryl groups.

4. A compound as claimed in Claim 2, wherein R₁ is a branched-, cyclic- or polycyclic-alkyloxycarbonyl group having 4 to 15 carbon atoms selected from among t-butyloxycarbonyl, adamantyloxycarbonyl and isobornyloxycarbonyl groups.

5. A compound as claimed in Claim 2, wherein R₁ is a substituted-benzyloxycarbonyl group having one or more substituents selected from among halogen atoms, nitro group and methoxy group.

6. A proteinase inhibitor which contains a compound represented by the general formula (1) shown in Claim 2 as an active ingredient.

7. A process for preparing a pharmaceutical composition by combining a compound as defined in claim as an active ingredient with one or more pharmaceutically acceptable carriers.

8. Use of a compound as defined in claim 2 for making a medicament, effective in inhibiting proteinases.

## Patentansprüche (Patentansprüche für folgende(n) Vertragsstaat(en): AT, BE, CH, DE, DK, FR, GB, IT, LI, LU, NL, SE)

1. Verbindung, dargestellt durch die folgende allgemeine Formel (1): 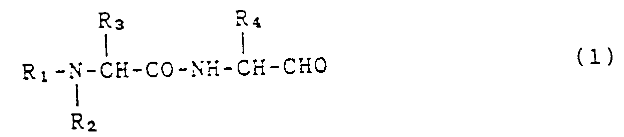 worin R₁ eine geradkettige oder verzweigte Acylgruppe mit 2 bis 10 Kohlenstoffatomen, eine verzweigte, zyklische oder polyzyklische Alkyloxycarbonylgruppe mit 4 bis 15 Kohlenstoffatomen, eine substituierte oder unsubstituierte Benzyloxycarbonylgruppe, eine 2,2,2-Trichloroethyloxycarbonylgruppe, eine 2-(Trimethylsilyl)ethyloxycarbonylgruppe, eine p-Toluolsulfonylgruppe, eine o-Nitrophenylsulfenylgruppe, eine Diphenylphosphonothioylgruppe, eine Triphenylmethylgruppe oder eine 2-Benzoyl-1-methylvinylgruppe ist; worin R₂ ein Wasserstoffatom ist; oder worin R₁ und R₂ zusammen eine Phthaloylgruppe bilden können, worin R₃ eine Isobutylgruppe oder eine n-Butylgruppe ist, und worin das oben erwähnte R₁ eine unsubstituierte Benzyloxycarbonxylgruppe sein kann, vorausgesetzt, daß R₃ eine n-Butylbruppe ist; und worin R₄ eine n-Butylgruppe ist.

2. Verbindung nach Anspruch 1,
worin R₁ eine geradkettige oder verzweigte Acylgruppe mit 2 bis 10 Kohlenstoffatomen ist, ausgewählt aus Octanoyl-, Caproyl- und Isovalerylgruppen.

3. Verbindung nach Anspruch 1,
worin R₁ eine verzweigte, zyklische oder polyzyklische Alkyloxycarbonylgruppe mit 4 bis 15 Kohlenstoffatomen ist, ausgewählt aus t-Butyloxycarbonyl-, Adamantyloxycarbonyl- und Isobornyloxycarbonylgruppen.

4. Verbindung nach Anspruch 1, worin R₁ eine substituierte Benzyloxycarbonylgruppe mit einem oder mehreren Substituenten ist, ausgewählt aus Halogenatomen, Nitrogruppe und Methoxygruppe.

5. Proteinaseinhibitor, umfassend eine Verbindung, dargestellt durch die allgemeine Formel (1), gezeigt in Anspruch 1, als einen aktiven Bestandteil.

6. Pharmazeutische Zusammensetzung, umfassend eine Verbindung nach Anspruch 1, als einen aktiven Bestandteil und einen oder mehrere pharmazeutisch akzeptable Träger.

7. Verwendung einer Verbindung nach Anspruch 1, zur Herstellung eines Medikamentes, wirkam bei der Inhibition von Proteinasen.

## Patentansprüche (Patentansprüche für folgende(n) Vertragsstaat(en): ES)

1. Verfahren zur Herstellung einer Verbindung mit der allgemeinen Formel (1): 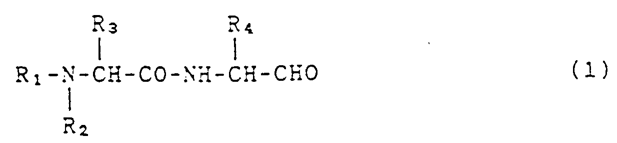 worin R₁ eine geradkettige oder verzweigte Acylgruppe mit 2 bis 10 Kohlenstoffatomen, eine verzweigte, zyklische oder polyzyklische Alkyloxycarbonylgruppe mit 4 bis 15 Kohlenstoffatomen, eine substituierte oder unsubstituierte Benzyloxycarbonylgruppe, eine 2,2,2-Trichloroethyloxycarbonylgruppe, eine 2-(Trimethylsilyl)ethyloxycarbonylgruppe, eine p-Toluolsulfonylgruppe, eine o-Nitrophenylsulfenylgruppe, eine Diphenylphosphonothioylgruppe, eine Triphenylmethylgruppe oder eine 2-Benzoyl-1-methylvinylgruppe ist; worin R₂ ein Wasserstoffatom ist; oder worin R₁ und R₂ zusammen eine Phthaloylgruppe bilden können, worin R₃ eine Isobutylgruppe oder eine n-Butylgruppe ist, und worin das oben erwähnte R₁ eine unsubstituierte Benzyloxycarbonxylgruppe sein kann, vorausgesetzt, daß R₃ eine n-Butylbruppe ist; und worin R₄ eine n-Butylgruppe ist, umfassend die folgenden Schritte:
Reduktion einer Verbindung, dargestellt durch die folgende allgemeine Formel (II): 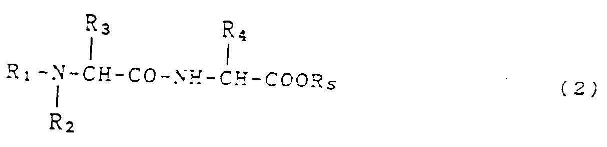 worin R₁, R₂, R₃, und R₄ wie oben definiert sind, und worin R₅ eine Niedrigalkylgruppe ist, durch Verwendung eines Reduktionsmittels in einem organischen Lösungsmittel, unter Erhalt einer Alkoholverbindung;
oder Reduktion einer Verbindung, dargestellt durch die folgende allgemeine Formel (III), 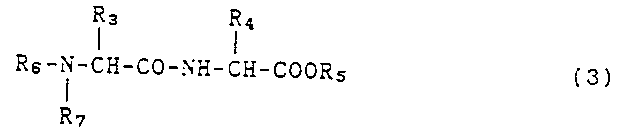 worin R₃, R₄ und R₅ wie oben im Hinblick auf die obige allgemeine Formel (2) definiet sind; worin R₆ eine Aminoschutzgruppe ist, die gegen eine Behandlung mit einer Base oder Reduktion unter Verwendung eines Reduktionsmittels stabil ist; und worin R₇ ein Wasserstoffatom ist; oder worin R₆ und R₇ zusammen eine Aminoschutzgruppe bilden, die gegen eine Behandlung mit einer Base oder Reduktion unter Verwendung eines Reduktionsmittels stabil ist; durch Verwendung eines Reduktionsmittels in einem organischen Lösungsmittel, unter Erhalt einer Alkoholverbindung,
Oxidation der Alkoholverbindung durch Verwendung eines Oxidationsmittels, unter Erhalt des Aldehydes.

2. Verfahren nach Anspruch 1, worin R₁ eine geradkettige oder verzweigte Acylgruppe mit 2 bis 10 Kohlenstoffatomen ist, ausgewählt aus Octanoyl-, Caproyl- und Isovalerylgruppen.

3. Verfahren nach Anspruch 1, worin R₁ eine verzweigte, zyklische oder polyzyklische Alkyloxycarbonylgruppe mit 4 bis 15 Kohlenstoffatomen ist, ausgewählt aus t-Butyloxycarbonyl-, Adamantyloxycarbonyl- und Isobornyloxycarbonylgruppen.

4. Verfahren nach Anspruch 1, worin R₁ eine substituierte Benzyloxycarbonylgruppe mit einem oder mehreren Substituenten ist, ausgewählt aus Halogenatomen, Nitrogruppe und Methoxygruppe.

5. Verfahren zur Herstellung einer pharmazeutischen Zusammensetzung durch Kombinieren einer Verbindung nach Anspruch 1 als einen aktiven Bestandteil mit einem oder mehreren pharmazeutisch akzeptablen Trägern.

6. Verfahren zur Herstellung einer pharmazeutischen Zusammensetzung nach Anspruch 5, zur Verwendung als ein Inhibitor von Proteinasen.

## Patentansprüche (Patentansprüche für folgende(n) Vertragsstaat(en): GR)

1. Verfahren zur Herstellung einer Verbindung mit der allgemeinen Formel (1): 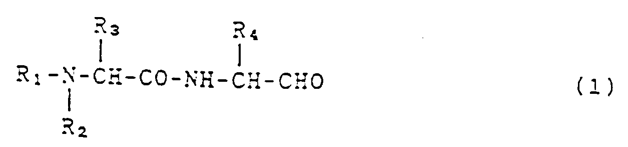 worin R₁ eine geradkettige oder verzweigte Acylgruppe mit 2 bis 10 Kohlenstoffatomen, eine verzweigte, zyklische oder polyzyklische Alkyloxycarbonylgruppe mit 4 bis 15 Kohlenstoffatomen, eine substituierte oder unsubstituierte Benzyloxycarbonylgruppe, eine 2,2,2-Trichloroethyloxycarbonylgruppe, eine 2-(Trimethylsilyl)ethyloxycarbonylgruppe, eine p-Toluolsulfonylgruppe, eine o-Nitrophenylsulfenylgruppe, eine Diphenylphosphonothioylgruppe, eine Triphenylmethylgruppe oder eine 2-Benzoyl-1-methylvinylgruppe ist; worin R₂ ein Wasserstoffatom ist; oder worin R₁ und R₂ zusammen eine Phthaloylgruppe bilden können, worin R₃ eine Isobutylgruppe oder eine n-Butylgruppe ist, und worin das oben erwähnte R₁ eine unsubstituierte Benzyloxycarbonxylgruppe sein kann, vorausgesetzt, daß R₃ eine n-Butylbruppe ist; und worin R₄ eine n-Butylgruppe ist, umfassend die folgenden Schritte:
Reduktion einer Verbindung, dargestellt durch die folgende allgemeine Formel (II): 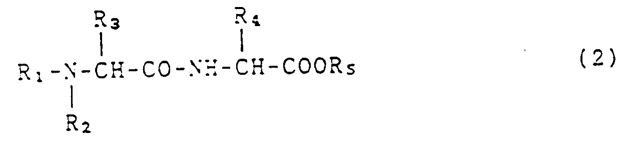 worin R₁, R₂, R₃, und R₄ wie oben definiert sind, und worin R₅ eine Niedrigalkylgruppe ist, durch Verwendung eines Reduktionsmittels in einem organischen Lösungsmittels, unter Erhalt einer Alkoholverbindung;
oder Reduktion einer Verbindung, dargestellt durch die folgende allgemeine Formel (III), 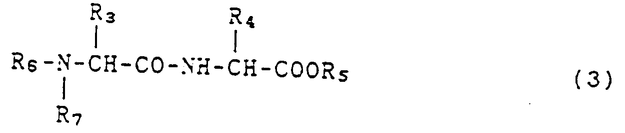 worin R₃, R₄ und R₅ wie oben im Hinblick auf die obige allgemeine Formel (2) definiert sind; worin R₆ eine Aminoschutzgruppe ist, die gegen eine Behandlung mit einer Base oder Reduktion unter Verwendung eines Reduktionsmittels stabil ist; und worin R₇ ein Wasserstoffatom ist; oder worin R₆ und R₇ zusammen eine Aminoschutzgruppe bilden, die gegen eine Behandlung mit einer Base oder Reduktion unter Verwendung eines Reduktionsmittels stabil ist; durch Verwendung eines Reduktionsmittels in einem organischen Lösungsmittel, unter Erhalt einer Alkoholverbindung,
Oxidation der Alkoholverbindung durch Verwendung eines Oxidationsmittels, unter Erhalt des Aldehydes.

2. Verbindung, dargestellt durch die folgende allgemeine Formel (1) 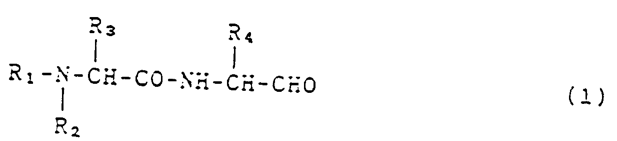 worin R₁ eine geradkettige oder verzweigte Acylgruppe mit 2 bis 10 Kohlenstoffatomen, eine verzweigte, zyklische oder polyzyklische Alkyloxycarbonylgruppe mit 4 bis 15 Kohlenstoffatomen, eine substituierte oder unsubstituierte Benzyloxycarbonylgruppe, eine 2,2,2-Trichloroethyloxycarbonylgruppe, eine 2-(Trimethylsilyl)ethyloxycarbonylgruppe, eine p-Toluolsulfonylgruppe, eine o-Nitrophenylsulfenylgruppe, eine Diphenylphosphonothioylgruppe, eine Triphenylmethylgruppe oder eine 2-Benzoyl-1-methylvinylgruppe ist; worin R₂ ein Wasserstoffatom ist; oder worin R₁ und R₂ zusammen eine Phthaloylgruppe bilden können, worin R₃ eine Isobutylgruppe oder eine n-Butylgruppe ist, und worin das oben erwähnte R₁ eine unsubstituierte Benzyloxycarbonxylgruppe sein kann, vorausgesetzt, daß R₃ eine n-Butylbruppe ist; und worin R₄ eine n-Butylgruppe darstellt.

3. Verbindung nach Anspruch 2,
worin R₁ eine geradkettige oder verzweigte Acylgruppe mit 2 bis 10 Kohlenstoffatomen ist, ausgewählt aus
Octanoyl-, Caproyl- und Isovalerylgruppen.

4. Verbindung nach Anspruch 2,
worin R₁ eine verzweigte, zyklische oder polyzyklische Alkyloxycarbonylgruppe mit 4 bis 15 Kohlenstoffatomen ist, ausgewählt aus t-Butyloxycarbonyl-, Adamantyloxycarbonyl- und Isobornyloxycarbonylgruppen.

5. Verbindung nach Anspruch 2, worin R₁ eine substituierte Benzyloxycarbonylgruppe mit einem oder mehreren Substituenten ist, ausgewählt aus Halogenatomen, Nitrogruppe und Methoxygruppe.

6. Proteinaseinhibitor, umfassend eine Verbindung, dargestellt durch die allgemeine Formel (1), gezeigt in Anspruch 2, als einen aktiven Bestandteil.

7. Verfahren zur Herstellung einer pharmazeutischen Zusammensetzung, durch Kombinieren einer Verbindung wie in Anspruch 2 definiert, als einen aktiven Bestandteil mit einem oder mehreren pharmazeutisch akzeptablen Trägern.

8. Verwendung einer Verbindung nach Anspruch 2, zur Herstellung eines Medikamentes, das bei der Inhibition von Proteinasen wirksam ist.

## Revendications (Revendications pour l'(les) Etat(s) contractant(s) suivant(s): AT, BE, CH, DE, DK, FR, GB, IT, LI, LU, NL, SE)

1. Composé de formule générale (1) 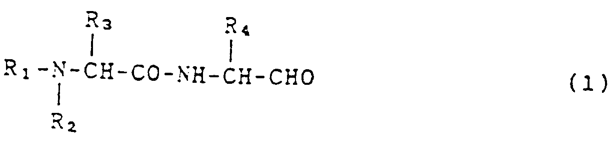 dans laquelle R₁ représente un groupe acyle à chaîne linéaire ou ramifiée possédant de 2 à 10 atomes de carbone, un groupe alkyloxycarbonyle ramifié, cyclique ou polycyclique possédant 4 à 15 atomes de carbone, un groupe benzyloxycarbonyle substitué ou non, un groupe 2,2,2-trichloroéthyloxycarbonyle, un groupe 2-(triméthylsilyl)éthyloxycarbonyle, un groupe p-toluènesulfonyle, un groupe o-nitrophénylsulfényle, un groupe diphénylphosphonothioyle, un groupe triphénylméthyle ou un groupe 2-benzoyl-1-méthylvinyle;
R₂ représente un atome d'hydrogène; ou R₁ et R₂ peuvent former ensemble un groupe phtaloyle;
R₃ représente un groupe isobutyle ou un groupe n-butyle et le radical R₁ précédemment mentionné peut être un groupe benzyloxycarbonyle non substitué sous réserve que R₃ soit un groupe n-butyle; et
R₄ représente un groupe n-butyle.

2. Composé selon la revendication 1 dans lequel R₁ représente un groupe acyle à chaîne linéaire ou ramifiée possédant de 2 à 10 atomes de carbone choisi parmi des groupes octanoyle, caproyle et isovaléryle.

3. Composé selon la revendication 1 dans lequel R₁ représente un groupe alkyloxycarbonyle ramifié, cyclique ou polycyclique possédant 4 à 15 atomes de carbone choisi parmi des groupes t-butyloxycarbonyle, adamantyloxycarbonyle et isobornyloxycarbonyle.

4. Composé selon la revendication 1 dans lequel R₁ représente un groupe benzyloxycarbonyle substitué, par un ou plusieurs substitutants choisis parmi les atomes d'halogène, un groupe nitro et un groupe méthoxy.

5. Inhibiteur des protéases qui contient un groupe représenté par la formule générale (1) présentée à la revendication 1 en tant que principe actif.

6. Composition pharmaceutique comprenant un composé tel que défini à la revendication 1 en tant que principe actif et un ou plusieurs supports acceptables d'un point de vue pharmaceutique

7. Utilisation d'un composé tel que défini à la revendication 1 pour préparer un médicament qui inhibe efficacement les protéases.

## Revendications (Revendications pour l'(les) Etat(s) contractant(s) suivant(s): ES)

1. Procédé de préparation d'un composé de formule générale (1) 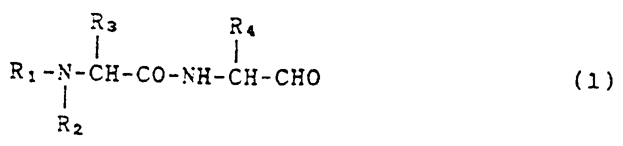 dans laquelle R₁ représente un groupe acyle à chaîne linéaire ou ramifiée possédant de 2 à 10 atomes de carbone, un groupe alkyloxycarbonyle ramifié, cyclique ou polycyclique possédant 4 à 15 atomes de carbone, un groupe benzyloxycarbonyle substitué ou non, un groupe 2,2,2-trichloroéthyloxycarbonyle, un groupe 2-(triméthylsilyl)éthyloxycarbonyle, un groupe p-toluènesulfonyle, un groupe o-nitrophénylsulfényle, un groupe diphénylphosphonothioyle, un groupe triphénylméthyle ou un groupe 2-benzoyl-1-méthylvinyle;
R₂ représente un atome d'hydrogène; ou R₁ et R₂ peuvent former ensemble un groupe phtaloyle;
R₃ représente un groupe isobutyle ou un groupe n-butyle et le radical R₁ précédemment mentionné peut être un groupe benzyloxycarbonyle non substitué sous réserve que R₃ soit un groupe n-butyle; et
R₄ représente un groupe n-butyle;
comprenant les étapes suivantes:
réduire une substance de formule générale (2) 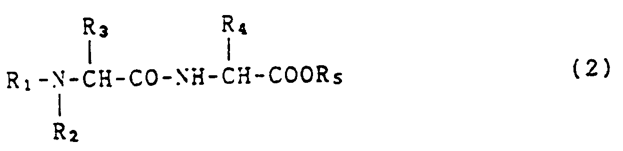 dans laquelle R₁, R₂, R₃ et R₄ sont tels que définis ci-dessus
et
R₅ représente un groupe alkyle inférieur,
à l'aide d'un agent réducteur dans un solvant organique de manière à donner un alcool;
ou réduire composé de formule générale (3) 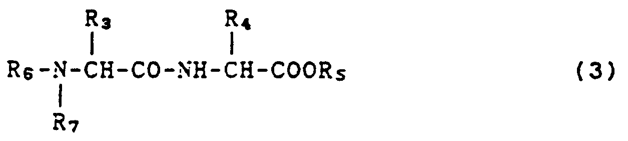 dans laquelle R₃, R₄ et R₅ sont tels que définis ci-dessus au sujet de la formule générale (2); et
R₆ représente un groupement protecteur du groupe amine qui est stable lors du traitement avec une base ou réduction à l'aide d'un agent réducteur, et R₇ représente un atome d'hydrogène; ou
R₆ et R₇ forment ensemble groupement protecteur du groupe amine qui est stable lors du traitement avec une base ou réduction à l'aide d'un agent réducteur;
à l'aide d'un agent réducteur dans un solvant organique de manière à donner un alcool;
l'oxydation dudit alcool de manière à donner l'aldéhyde.

2. Procédé selon la revendication 1 dans lequel R1 représente un groupe acyle à chaîne linéaire ou ramifiée possédant de 2 à 10 atomes de carbone choisi parmi des groupes octanoyle, caproyle et isovaléryle.

3. Procédé selon la revendication 1 dans lequel R1 représente un groupe alkyloxycarbonyle ramifié, cyclique ou polycyclique possédant 4 à 15 atomes de carbone choisi parmi des groupes t-butyloxycarbonyle, adamantyloxycarbonyle ou isobornyloxycarbonyle.

4. Procédé selon la revendication 1 dans lequel R1 représente un groupe benzyloxycarbonyle substitué par un ou plusieurs substitutants choisis parmi les atomes d'halogènes, un groupe nitro et un groupe méthoxy.

5. Procédé de préparation d'une composition pharmaceutique associant comprenant un composé tel que défini à la revendication 1 en tant que principe actif et un ou plusieurs supports acceptables d'un point de vue pharmaceutique

6. Procédé de préparation d'une composition pharmaceutique selon la revendication 5 destinée à être utilisée comme inhibiteur des protéases.

## Revendications (Revendications pour l'(les) Etat(s) contractant(s) suivant(s): GR)

1. Procédé de préparation d'un composé de formule générale (1) 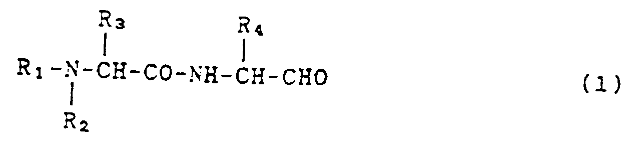 dans laquelle R₁ représente un groupe acyle à chaîne linéaire ou ramifiée possédant de 2 à 10 atomes de carbone, un groupe alkyloxycarbonyle ramifié, cyclique ou polycyclique possédant 4 à 15 atomes de carbone, un groupe benzyloxycarbonyle substitué ou non, un groupe 2,2,2-trichloroéthyloxycarbonyle, un groupe 2-(triméthylsilyl)éthyloxycarbonyle, un groupe p-toluènesulfonyle, un groupe o-nitrophénylsulfényle, un groupe diphénylphosphonothioyle, un groupe triphénylméthyle ou un groupe 2-benzoyl-1-méthylvinyle;
R₂ représente un atome d'hydrogène; ou R₁ et R₂ peuvent former ensemble un groupe phtaloyle;
R₃ représente un groupe isobutyle ou un groupe n-butyle et le radical R₁ précédemment mentionné peut être un groupe benzyloxycarbonyle non substitué sous réserve que R₃ soit un groupe n-butyle; et
R₄ représente un groupe n-butyle;
comprenant les étapes suivantes:
réduire une substance de formule générale (2) 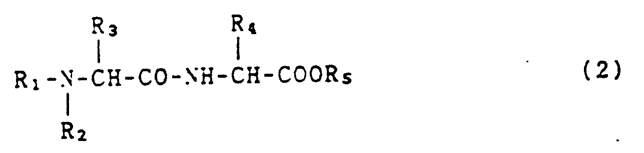 dans laquelle R₁, R₂, R₃ et R₄ sont tels que définis ci-dessus
et
R₅ représente un groupe alkyle inférieur;
à l'aide d'un agent réducteur dans un solvant organique de manière à donner un alcool;
ou réduire composé de formule générale (3) 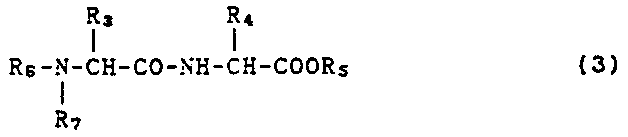 dans laquelle R₃, R₄ et R₅ sont tels que définis ci-dessus au sujet de la formule générale (2); et
R₆ représente un groupement protecteur du groupe amine qui est stable lors du traitement avec une base ou réduction à l'aide d'un agent réducteur; et R₇ représente un atome d'hydrogène; ou
R₆ et R₇ forment ensemble groupement protecteur du groupe amine qui est stable lors du traitement avec une base ou réduction à l'aide d'un agent réducteur;
à l'aide d'un agent réducteur dans un solvant organique de manière à donner un alcool; et
l'oxydation dudit alcool de manière à donner l'aldéhyde.

2. Composé de formule générale (1) 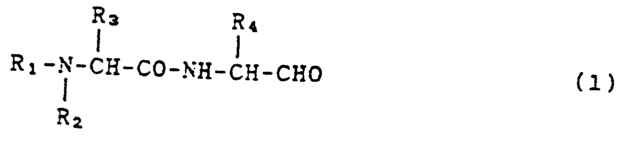 dans laquelle R₁ représente un groupe acyle à chaîne linéaire ou ramifiée possédant de 2 à 10 atomes de carbone, un groupe alkyloxycarbonyle ramifié, cyclique ou polycyclique possédant 4 à 15 atomes de carbone, un groupe benzyloxycarbonyle substitué ou non, un groupe 2,2,2-trichloroéthyloxycarbonyle, un groupe 2-(triméthylsilyl)éthyloxycarbonyle, un groupe p-toluènesulfonyle, un groupe o-nitrophénylsulfényle, un groupe diphénylphosphonothioyle, un groupe triphénylméthyle ou un groupe 2-benzoyl-1-méthylvinyle;
R₂ représente un atome d'hydrogène; ou R₁ et R₂ peuvent former ensemble un groupe phtaloyle;
R₃ représente un groupe isobutyle ou un groupe n-butyle et le radical R₁ précédemment peut être un groupe benzyloxycarbonyle non substitué sous réserve que R₃ soit un groupe n-butyle; et
R₄ représente un groupe n-butyle.

3. Composé selon la revendication 2 dans lequel R₁ représente un groupe acyle à chaîne linéaire ou ramifiée possédant de 2 à 10 atomes de carbone choisi parmi des groupes octanoyle, caproyle et isovaléryle.

4. Composé selon la revendication 2 dans lequel R₁ représente un groupe alkyloxycarbonyle ramifié, cyclique ou polycyclique possédant 4 à 15 atomes de carbone choisi parmi des groupes t-butyloxycarbonyle, adamantyloxycarbonyle ou isobornyloxycarbonyle.

5. Composé selon la revendication 2 dans lequel R₁ représente un groupe benzyloxycarbonyle substitué par un ou plusieurs substitutants choisis parmi les atomes d'halogènes, un groupe nitro et un groupe méthoxy.

6. Inhibiteur des protéases qui contient un groupe représenté par la formule générale (1) présentée à la revendication 2 en tant que principe actif.

7. Procédé de préparation d'une composition pharmaceutique associant comprenant un composé tel que defini à la revendication 2 en tant que principe actif et un en plusieurs supports acceptables d'un point de vue pharmaceutique.

8. Utilisation d'un composé tel que defini a la revendication 2 pour préparer un medicament qui inhibe efficacement les protéases.
